# EUROPEAN PATENT APPLICATION

(11) **EP 3 649 960 A1**
(43) Date of publication of application: **13.05.2020**
(21) Application number: 18828949.0
(22) Date of filing: 18.04.2018
(51) Int. Cl.: A61B 8/14, A61B 8/13

(54) **ACOUSTIC WAVE DIAGNOSTIC DEVICE AND METHOD FOR OPERATING ACOUSTIC WAVE DIAGNOSTIC DEVICE**

(30) Priority: 04.07.2017 JP 2017131417; 22.08.2017 JP 2017159749
(71) Applicant: FUJIFILM Corporation, Tokyo 106-8620 (JP)
(72) Inventor: IMAI, Yoshiro, Ashigarakami-gun Kanagawa 258-8538 (JP)
(74) Representative: Klunker IP Patentanwälte PartG mbB
(86) International application number: PCT/JP2018/015942
(87) International publication number: WO 2019/008867

(57) **Abstract**

An acoustic wave diagnostic apparatus (1) includes: an image display unit (14) that displays an acquired acoustic wave image; a measurement item designation receiving unit (28) that receives designation of a measurement item relevant to a measurement target; a detection measurement algorithm setting unit (29) that sets a detection measurement algorithm based on the received measurement item; a position designation receiving unit (30) that receives designation of a position of the measurement target on an acoustic wave image (Ib) displayed on the image display unit (14); and a measurement unit (31) that, in a case where the designation of the position is received, detects the measurement target based on the received position and the detection measurement algorithm and performs measurement for the detected measurement target.

## Description

### BACKGROUND OF THE INVENTION

### 1. Field of the Invention

The present invention relates to a medical image diagnostic apparatus using an acoustic wave, and relates to an acoustic wave diagnostic apparatus and an operation method of an acoustic wave diagnostic apparatus for measuring an organ, a lesion, and the like on an acoustic wave image displayed on a display device.

### 2. Description of the Related Art

In recent years, medical acoustic wave diagnostic apparatuses generally have a measurement function for measuring the length, size, area, and the like of various organs, lesions, and the like included in an acquired acoustic wave image. In order to measure a measurement target, an operator usually operates a caliper, that is, a cursor using an input device for inputting coordinates, such as a track pad, a track ball, and a mouse, to set a measurement point, a region of interest, and the like on a display image.

JP2010-240198A discloses an ultrasound diagnostic apparatus that allows the operator to concentrate on diagnosis without being confused by the operation by automatically determining the optimal measurement item according to the type of measurement target part, an image mode under measurement, and the setting order of measurement points input by the operator. JP2010-148811A discloses a technique for solving a problem that it is difficult to see an ultrasound diagnostic image or a measurement result by preventing contamination due to fingerprints adhering to display means by separately comprising display means for displaying an ultrasound diagnostic image and a display and reception means for giving an instruction for measurement processing.

### SUMMARY OF THE INVENTION

In both JP2010-240198A and JP2010-148811A, since measurement points are input only by a manual input operation, it takes time or effort to align the measurement points with the measurement target. In addition, the operator may have variations in the input of measurement points, and the measurement results may also vary.

The present invention has been made in view of such problems, and it is an object of the present invention to provide an acoustic wave diagnostic apparatus and an operation method of an acoustic wave diagnostic apparatus capable of measuring a measurement target quickly and easily by reducing the time or effort required for an operator to input measurement points and capable of performing measurement with reduced variations by the operator.

An acoustic wave diagnostic apparatus according to an aspect of the present invention comprises: an image display unit that displays an acquired acoustic wave image; a measurement item designation receiving unit that receives designation of a measurement item relevant to a measurement target; a detection measurement algorithm setting unit that sets a detection measurement algorithm based on the measurement item received by the measurement item designation receiving unit; a position designation receiving unit that receives designation of a position of the measurement target on the acoustic wave image displayed on the image display unit; and a measurement unit that, in a case where the designation of the position is received by the position designation receiving unit, detects the measurement target based on the received position and the detection measurement algorithm set by the detection measurement algorithm setting unit and performs measurement for the detected measurement target.

In the present invention, the "acoustic wave" is a term including an ultrasound wave and a photoacoustic wave. The "detection measurement algorithm" is an algorithm including an algorithm for detection and an algorithm for measurement. The "measurement item relevant to the measurement target" is an item that can indicate the measurement target and the measurement content. For example, the measurement item includes the name of an organ and the measurement content regarding the organ, such as the size or the length. In addition, in a case where the measurement item is, for example, only the name of an organ, the measurement content regarding the organ may be associated with the name of the organ.

In the acoustic wave diagnostic apparatus according to an aspect of the present invention, the measurement unit may determine a position of a detection range in which the detection is performed based on the position received by the position designation receiving unit.

In the present invention, the "detection range" means the size or shape of a region where the measurement unit performs detection, and the "position of the detection range" means a position where the region is present.

In the acoustic wave diagnostic apparatus according to an aspect of the present invention, the measurement unit may determine a measurement position at which the measurement is performed based on the position received by the position designation receiving unit.

In the present invention, the "measurement position" means a place where measurement points are arranged. As for the "measurement point", in the case of measuring the distance between two points in the measurement target, the two points are referred to as measurement points.

The acoustic wave diagnostic apparatus according to an aspect of the present invention acoustic may further comprise a detection condition setting unit that sets condition, under which the detection for the measurement target is performed, based on at least one of the measurement item or the position received by the position designation receiving unit, and the measurement unit may perform detection based on the condition set by the detection condition setting unit.

In the acoustic wave diagnostic apparatus according to an aspect of the present invention, the detection condition setting unit may determine at least one of a position of a detection range, a size of the detection range, a detection accuracy, or a detection order as the condition for performing the detection.

The acoustic wave diagnostic apparatus according to an aspect of the present invention may further comprise a measurement point arrangement rule setting unit that sets an arrangement rule of measurement points in the measurement target based on the measurement item, and the measurement unit may perform measurement by arranging measurement points based on the measurement point arrangement rule set by the measurement point arrangement rule setting unit.

In the acoustic wave diagnostic apparatus according to an aspect of the present invention, the measurement item designation receiving unit may receive designation of at least one of a name of an organ, a name of a lesion, a name of an organ and measurement content of the organ, or a name of a lesion and measurement content of the lesion as a measurement item relevant to the measurement target.

In the acoustic wave diagnostic apparatus according to an aspect of the present invention, the acoustic wave image may be an ultrasound image.

In the acoustic wave diagnostic apparatus according to an aspect of the present invention, the acoustic wave image may be a photoacoustic wave image.

An operation method of an acoustic wave diagnostic apparatus according to an aspect of the present invention is an operation method of an acoustic wave diagnostic apparatus comprising an image display unit, a measurement item designation receiving unit, a detection measurement algorithm setting unit, a position designation receiving unit, and a measurement unit. The operation method of an acoustic wave diagnostic apparatus according to an aspect of the present invention comprises: causing the image display unit to display an acquired acoustic wave image; causing the measurement item designation receiving unit to receive designation of a measurement item relevant to a measurement target; causing the detection measurement algorithm setting unit to set a detection measurement algorithm based on the measurement item received by the measurement item designation receiving unit; causing the position designation receiving unit to receive designation of a position of the measurement target on the acoustic wave image displayed on the image display unit; and in a case where the designation of the position is received by the position designation receiving unit, causing the measurement unit to detect the measurement target based on the received position and the detection measurement algorithm set by the detection measurement algorithm setting unit and perform measurement for the detected measurement target.

According to the acoustic wave diagnostic apparatus and the operation method of an acoustic wave diagnostic apparatus of the present invention, there are comprised the image display unit that displays an acquired acoustic wave image; the measurement item designation receiving unit that receives designation of a measurement item relevant to a measurement target; the detection measurement algorithm setting unit that sets a detection measurement algorithm based on the measurement item received by the measurement item designation receiving unit; the position designation receiving unit that receives designation of a position of the measurement target on the acoustic wave image displayed on the image display unit; and the measurement unit that, in a case where the designation of the position is received by the position designation receiving unit, detects the measurement target based on the received position and the detection measurement algorithm set by the detection measurement algorithm setting unit and performs measurement for the detected measurement target. Therefore, the measurement unit automatically detects and measures the measurement target simply by designating the approximate position of the measurement target on the acoustic wave image displayed on the image display unit by the operator. Therefore, it is possible to measure the measurement target quickly and easily by reducing the time or effort required for the operator to input measurement points and to perform measurement with reduced variations by the operator.

### BRIEF DESCRIPTION OF THE DRAWINGS

Fig. 1 is a perspective view showing an acoustic wave image capturing apparatus comprising an acoustic wave diagnostic apparatus according to a first embodiment of the present invention.
Fig. 2 is a schematic diagram showing the overall configuration of the acoustic image capturing apparatus shown in Fig. 1.
Fig. 3 is a diagram showing an example of the display of an ultrasound image.
Fig. 4 is a diagram showing an example of the display of a list of measurement items on the ultrasound image.
Fig. 5 is a diagram showing an example of selection in the list of measurement items displayed on the ultrasound image.
Fig. 6 is a diagram showing an example of designation of a position on the ultrasound image.
Fig. 7 is a diagram showing an example of a measurement result displayed on the ultrasound image.
Fig. 8 is a flowchart showing a method of a series of processes of the acoustic wave diagnostic apparatus shown in Fig. 2.
Fig. 9 is a perspective view showing an acoustic wave image capturing apparatus comprising an acoustic wave diagnostic apparatus according to a second embodiment of the present invention.
Fig. 10 is a schematic diagram showing the overall configuration of the acoustic wave image capturing apparatus shown in Fig. 9.
Fig. 11 is a schematic diagram showing the overall configuration of an acoustic wave image capturing apparatus of a third embodiment comprising an acoustic wave diagnostic apparatus according to the second embodiment of the present invention.
Fig. 12 is a diagram illustrating processing by the acoustic wave diagnostic apparatus shown in Fig. 11.
Fig. 13 is a diagram showing an example of the size of a detection range on the ultrasound image.
Fig. 14 is a diagram showing another example of the size of the detection range on the ultrasound image.
Fig. 15 is a diagram showing an example of the position of a detection range on the ultrasound image.
Fig. 16 is a diagram showing another example of the position of the detection range on the ultrasound image.
Fig. 17 is a diagram illustrating the detection accuracy within the detection range.
Fig. 18 is a diagram showing an example of the detection order on the ultrasound image.
Fig. 19 is a diagram showing another example of the detection order on the ultrasound image.
Fig. 20 is a flowchart showing a method of a series of processes of the acoustic wave diagnostic apparatus shown in Fig. 11.
Fig. 21 is a schematic diagram showing the overall configuration of an acoustic wave image capturing apparatus of a fourth embodiment comprising an acoustic wave diagnostic apparatus according to the third embodiment of the present invention.
Fig. 22 is a diagram showing an example of the arrangement of measurement points.
Fig. 23 is a diagram showing another example of the arrangement of measurement points.
Fig. 24 is an example of the arrangement of measurement points in different measurement items.
Fig. 25 is a flowchart showing a method of a series of processes of the acoustic wave diagnostic apparatus shown in Fig. 21.

### DESCRIPTION OF THE PREFERRED EMBODIMENTS

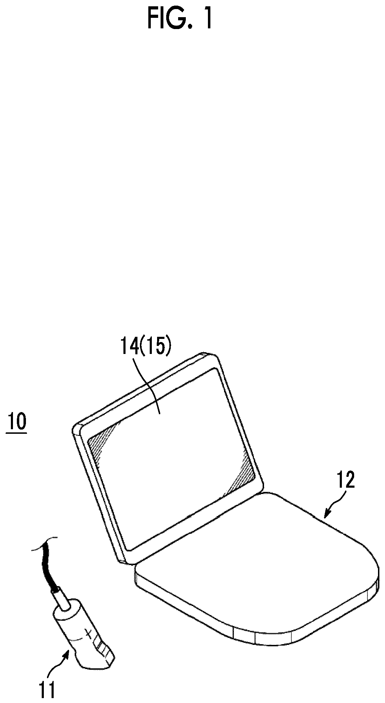

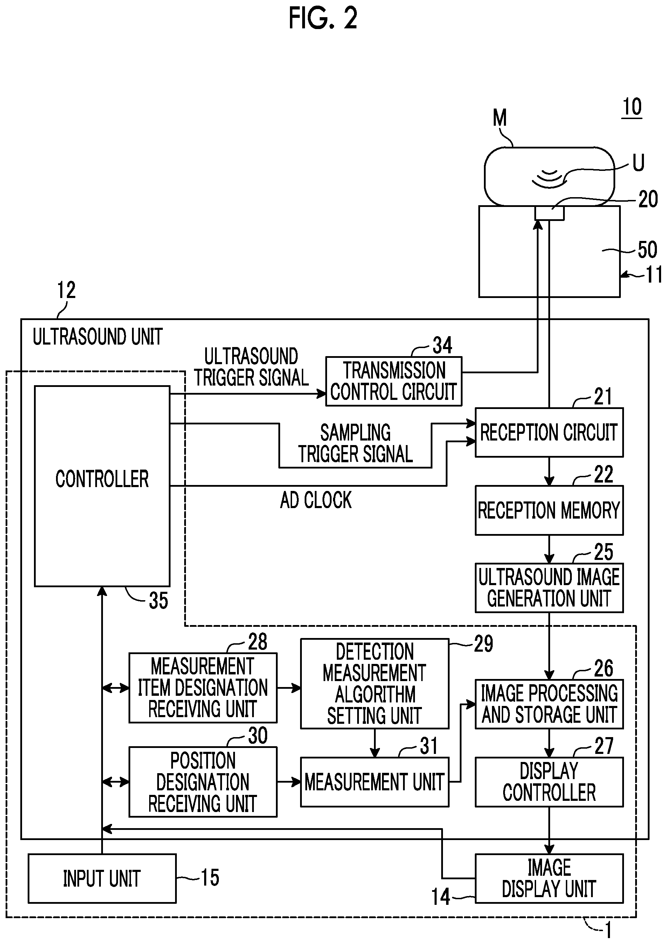

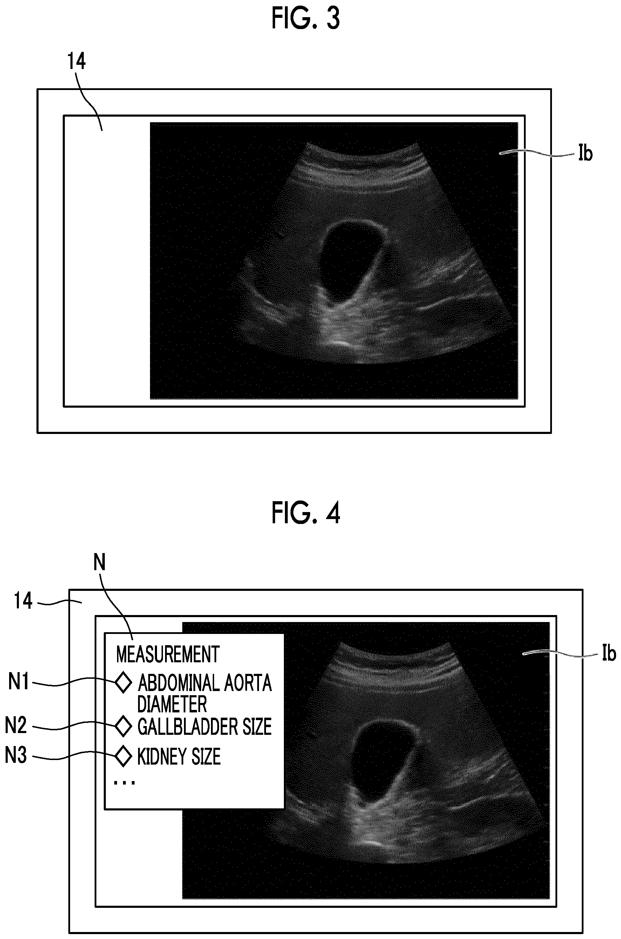

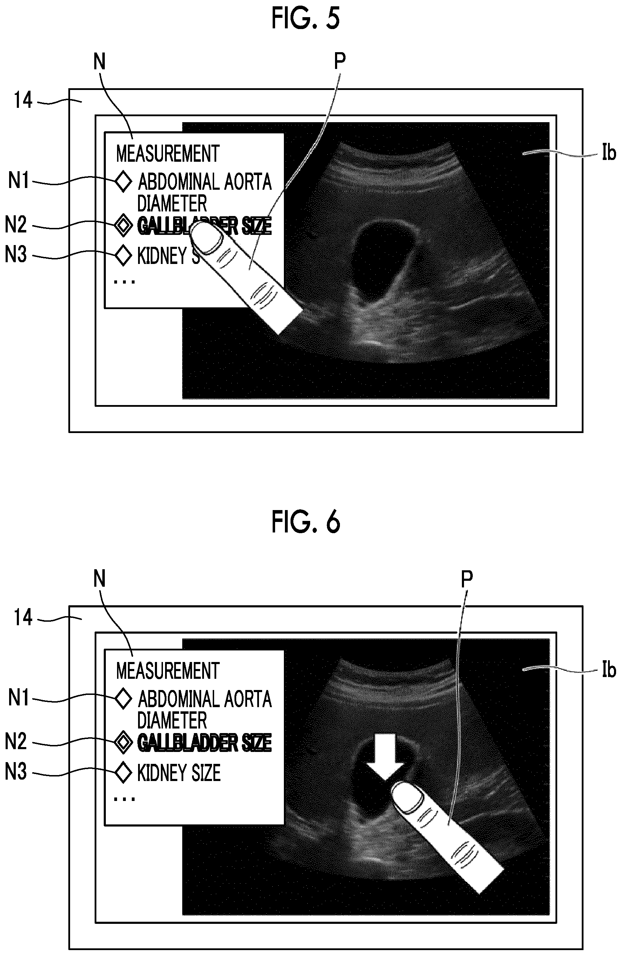

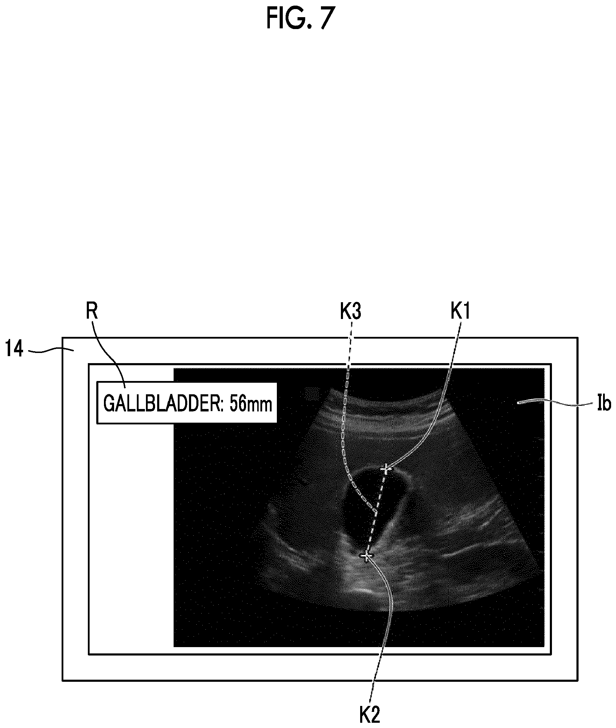

Hereinafter, an acoustic wave image capturing apparatus 10 comprising an acoustic wave diagnostic apparatus 1 according to a first embodiment of the present invention will be described in detail with reference to the diagrams. Fig. 1 is a perspective view showing the acoustic wave image capturing apparatus 10 comprising the acoustic wave diagnostic apparatus 1 according to the first embodiment of the present invention. Fig. 2 is a schematic diagram showing the overall configuration of the acoustic wave image capturing apparatus 10 shown in Fig. 1. Fig. 3 is a diagram showing an example of the display of an ultrasound image Ib. Fig. 4 is a diagram showing an example of the display of a list N of measurement items on the ultrasound image Ib. Fig. 5 is a diagram showing an example of selection in the list N of measurement items displayed on the ultrasound image Ib. Fig. 6 is a diagram showing an example of designation of a position on the ultrasound image Ib. Fig. 7 is a diagram showing an example of a measurement result R displayed on the ultrasound image Ib.

As an example, the acoustic wave image capturing apparatus 10 according to the present embodiment has only a function of generating an ultrasound image based on a reflected ultrasound wave detection signal. The acoustic wave diagnostic apparatus according to the embodiment of the present invention may be mounted in an apparatus having both a function of generating a photoacoustic image based on a photoacoustic signal and a function of generating an ultrasound image based on a reflected ultrasound wave detection signal, or may be mounted in an apparatus having only a function of generating a photoacoustic image. The acoustic wave diagnostic apparatus according to the embodiment of the present invention can be mounted in any apparatus that can receive or store image data of at least one of a photoacoustic image or an ultrasound image of a two-dimensional image and display the received or stored image data even though the apparatus does not have any of the above-described functions.

As an example, the acoustic wave image capturing apparatus 10 is configured as a so-called portable notebook computer type apparatus, as shown in Fig. 1. Although the acoustic wave image capturing apparatus 10 of the present embodiment is of a notebook computer type, the present invention is not limited thereto, and may be of a tablet type or the like. As shown in Figs. 1 and 2, the acoustic wave image capturing apparatus 10 comprises a probe 11 that is an ultrasound probe, an ultrasound unit 12, an image display unit 14, and an input unit 15. The ultrasound unit 12 is housed in the housing of Fig. 1. More specifically, the image display unit 14 is an image display screen including, for example, a liquid crystal display device, and is configured by a touch panel to which direct input can be performed by the operator. The image display unit 14 of the present embodiment also functions as the input unit 15. The image display unit 14 and the input unit 15 are configured to be able to display a color image. Hereinafter, the components of the acoustic wave image capturing apparatus 10 will be sequentially described.

The probe 11 has a function of emitting ultrasound waves toward a subject M that is a living body, for example. In Fig. 2, the shape of the probe 11 is schematically shown. The probe 11 has a function of detecting an acoustic wave U propagating through the subject M. The probe 11 emits (transmits) ultrasound waves (acoustic waves) to the subject M, and detects (receives) reflected ultrasound waves (reflected acoustic waves) that are returned by reflection from the subject M.

In this specification, the "acoustic wave" includes an ultrasound wave or a photoacoustic wave, and the "ultrasound wave" means an elastic wave transmitted by the probe and its reflected wave (reflected ultrasound wave). The acoustic wave emitted from the probe 11 is not limited to the ultrasound wave, and an acoustic wave having an audible frequency may be used as long as an appropriate frequency can be selected according to an examination target, measurement conditions, and the like.

The probe 11 is configured as, for example, a sector scanning probe, a linear scanning probe, or a convex scanning probe. The type of probe used for acquisition of an acoustic wave image is appropriately selected according to an imaging part and the like. The probe 11 has a transducer array 20 that is an acoustic wave detector, and the transducer array 20 is housed in a housing 50. In the present embodiment, the transducer array 20 also functions as an ultrasound wave transmission element. The transducer array 20 is connected to a circuit for transmitting ultrasound waves and the like through wiring lines (not shown).

The transducer array 20 has a plurality of ultrasound transducers arranged to be parallel in one direction. The ultrasound transducer is an electroacoustic transducer. The ultrasound transducer is a piezoelectric element formed of, for example, piezoelectric ceramics. Alternatively, the ultrasound transducer may be a piezoelectric element formed of a polymer film, such as polyvinylidene difluoride (PVDF). The ultrasound transducer converts the received acoustic wave U into an electrical signal.

Although an example of the transducer array 20 in which a plurality of ultrasound transducers are arranged in a one-dimensional manner has been described above, the transducer array 20 is not limited thereto. In the transducer array 20, a plurality of ultrasound transducers may be arranged in a two-dimensional manner.

In the transducer array 20, in a case where an alternating voltage is applied to the ultrasound transducer, the ultrasound transducer generates an ultrasound wave having a frequency corresponding to the frequency of the alternating voltage, and the ultrasound wave is transmitted from the transducer array 20. In addition, transmission and reception of ultrasound waves may be separated from each other. That is, for example, ultrasound waves may be transmitted from a position different from the probe 11, and reflected ultrasound waves of the transmitted ultrasound waves may be received by the probe 11.

The ultrasound unit 12 has a reception circuit 21, a reception memory 22, an ultrasound image generation unit 25, an image processing and storage unit 26, a display controller 27, a measurement item designation receiving unit 28, a detection measurement algorithm setting unit 29, a position designation receiving unit 30, a measurement unit 31, a transmission control circuit 34, and a controller 35. In the present embodiment, the acoustic wave diagnostic apparatus 1 that is an embodiment of the present invention is configured by the image display unit 14, the input unit 15, the image processing and storage unit 26, the display controller 27, the measurement item designation receiving unit 28, the detection measurement algorithm setting unit 29, the position designation receiving unit 30, the measurement unit 31, and the controller 35.

The controller 35 controls each unit of the acoustic wave diagnostic apparatus 1, and controls each unit of the acoustic wave image capturing apparatus 10. In the case of acquiring an ultrasound image, the controller 35 transmits an ultrasound trigger signal for instructing the transmission control circuit 34 to transmit ultrasound waves. In a case where the ultrasound trigger signal is received, the transmission control circuit 34 causes the probe 11 to transmit ultrasound waves. The controller 35 transmits a sampling trigger signal to the reception circuit 21 according to the timing of ultrasound wave transmission, thereby starting the sampling of the reflected ultrasound wave signal.

The reception circuit 21 receives the reflected ultrasound wave detection signal output from the transducer array 20 of the probe 11, and stores the received detection signal in the reception memory 22. Typically, the reception circuit 21 is configured to include a low noise amplifier, a variable gain amplifier, a low pass filter, and an AD converter. The reflected ultrasound wave detection signal output from the probe 11 is amplified by the low noise amplifier and then the gain is adjusted according to the depth by the variable gain amplifier, and high-frequency components are cut by the low pass filter and then conversion into a digital signal is performed by the AD converter and the digital signal is stored in the reception memory 22. The reception circuit 21 is configured by, for example, one integrated circuit (IC). The above-described low pass filter is provided to prevent aliasing noise from occurring at the time of AD conversion. The cut-off frequency of the low pass filter is generally set to a frequency that is about half the sampling frequency of AD conversion. Specifically, the cut-off frequency of the low pass filter is set to about 10 MHz to 30 MHz.

A digitized reflected ultrasound wave detection signal is stored in the reception memory 22. More specifically, the reflected ultrasound wave detection signal is a signal obtained by detecting a reflected acoustic wave that is a reflected wave of an ultrasound wave, which is an acoustic wave emitted toward the subject M, in the subject M.

The ultrasound image generation unit 25 generates an ultrasound image (tomographic image) by reconstructing the reflected ultrasound wave detection signal received from the reception memory 22. Specifically, the ultrasound image generation unit 25 adds a reflected ultrasound wave detection signal based on a signal output from each ultrasound transducer with a delay time corresponding to the position of each ultrasound transducer of the transducer array 20, thereby generating a reflected ultrasound wave detection signal for one line (delay addition method). The ultrasound image generation unit 25 may perform reconstruction using a circular back projection (CBP) method instead of the delay addition method. Alternatively, the ultrasound image generation unit 25 may perform reconstruction using a Hough transform method or a Fourier transform method. The reconstructed reflected ultrasound wave detection signals for a plurality of lines are subjected to signal processing, such as detection processing and logarithmic conversion processing, and then are transmitted to the display controller 27 through the image processing and storage unit 26 as signals for displaying an ultrasound image (tomographic image) regarding a cross section of the subject M.

The image processing and storage unit 26 performs various kinds of processing for image quality improvement, such as brightness correction, gradation correction, sharpness correction, and color correction, on the image data of the ultrasound image. The image processing and storage unit 26 of the present embodiment also functions as a storage unit that stores various databases and a storage unit that stores, as image data, a signal for displaying the image generated by the ultrasound image generation unit 25. Although the image processing and storage unit 26 of the acoustic wave diagnostic apparatus 1 and the acoustic wave image capturing apparatus 10 of the present embodiment comprises both an image processing function and a function as a storage unit, the acoustic wave diagnostic apparatus according to the embodiment of the present invention may have only a function as a storage unit without having an image processing function.

The display controller 27 displays the ultrasound image Ib on the image display unit 14 as shown in Fig. 3 based on the above-described signal for displaying the ultrasound image.

The measurement item designation receiving unit 28 receives designation of a measurement item relevant to the measurement target. Specifically, in a case where the measurement function is started on a user interface (UI) application by the operator, the list N of measurement items relevant to the measurement target is displayed on the image display unit 14 as shown in Fig. 4. In each measurement item displayed in the list N of measurement items, the name of an organ and the measurement content regarding the organ are described. For example, an abdominal aorta diameter N1, a gallbladder size N2, a kidney size N3, a gallbladder wall thickness, and the like are displayed. Although the name of the organ and the measurement content regarding the organ are displayed as measurement items relevant to the measurement target in the present embodiment, the present invention is not limited thereto, and only the name of the organ may be displayed. In addition, without being limited to the name of the organ, only items relevant to the names or abnormalities of lesions such as tumors, cysts, and bleeding may be displayed, or the names of lesions and the measurement content regarding the lesions may be displayed, or an item regarding abnormality and the measurement content regarding the item regarding abnormality may be displayed. The display content can be appropriately changed. For example, in the case of displaying only the name of an organ, the displayed name of the organ is associated with the measurement content, such as measuring the diameter, measuring the size, or measuring the length. Specifically, an association table is stored in the image processing and storage unit 26, and the measurement content is selected based on the table in a case where the name of an organ is designated.

In a case where the list N of measurement items is displayed on the image display unit 14 as shown in Fig. 4, the operator selects, for example, the gallbladder size N2 as a desired measurement item from the list N of measurement items using a finger P as shown in Fig. 5. The measurement item designation receiving unit 28 receives the gallbladder size N2 selected by the operator as a measurement item. Although the measurement item designation receiving unit 28 of the present embodiment receives a selection item selected by a touch panel, the present invention is not limited thereto. For example, an input device, such as a mouse, a track pad, or a track ball, may be used as an input unit, and a selection item selected by the input unit may be received.

The detection measurement algorithm setting unit 29 sets a detection algorithm and a measurement algorithm based on the measurement item received by the measurement item designation receiving unit 28. In general, an algorithm for detecting a measurement target on an image differs depending on the type of organ, lesion, or the like. In addition, the algorithm for measuring a measurement target on the image also differs depending on the measurement content, such as measuring the diameter, measuring the size, or measuring the length. The detection measurement algorithm setting unit 29 stores an algorithm corresponding to each measurement target and an algorithm corresponding to each measurement content as an association table, and sets a detection measurement algorithm with reference to the above-described association table in a case where the measurement item designation receiving unit 28 receives a measurement item. As the detection measurement algorithm, a known algorithm that is generally used can be used. The algorithm referred to herein defines a procedure (calculation procedure) for achieving a certain purpose (detection or measurement). For example, the algorithm referred to herein is implemented as a software program in an apparatus and is executed by a processor.

For example, for a measurement target detection algorithm, there is a method in which typical pattern data is stored in advance as a template, a pattern data similarity is calculated while searching for an image with a template, and it is considered that a target is present in a place where the similarity is equal to or greater than a predetermined threshold value and is the maximum. For the calculation of the similarity, in addition to simple template matching, for example, a machine learning method described in Csurka et al.: Visual Categorization with Bags of Keypoints, Proc. of ECCV Workshop on Statistical Learning in Computer Vision, pp. 59-74 (2004) or a general image recognition method using deep learning described in Krizhevsk et al.: ImageNet Classification with Deep Convolutional Neural Networks, Advances in Neural Information Processing Systems 25, pp.1106-1114 (2012) can be used.

The position designation receiving unit 30 receives designation of the position of a measurement target on the ultrasound image Ib displayed on the image display unit 14. Specifically, in a case where the measurement item received by the measurement item designation receiving unit 28 is the gallbladder size N2, the operator desires to measure the size of the gallbladder. Therefore, the measurement target is the gallbladder. Then, as shown in Fig. 6, the operator designates a position where the operator himself or herself thinks that the gallbladder is present on the ultrasound image Ib, for example, the tip of the arrow in Fig. 6 using the finger P. The position designation receiving unit 30 receives the position designated by the operator as a measurement target position.

In a case where the designation of the position of the measurement target is received by the position designation receiving unit 30, the measurement unit 31 detects the measurement target based on the received position and the detection measurement algorithm set by the detection measurement algorithm setting unit 29, and performs measurement for the detected measurement target. The measurement unit 31 determines the position of a detection range for detecting the measurement target based on the received position, and detects the inside of the detection range at the determined position. The size of the detection range is set in the measurement unit 31 in advance, but can be changed by the operator.

Specifically, in a case where the measurement item received by the measurement item designation receiving unit 28 is the gallbladder size N2, the measurement unit 31 first detects the gallbladder on the ultrasound image Ib within the detection range based on the detection algorithm set by the detection measurement algorithm setting unit 29. The measurement unit 31 detects the accurate position and region of the gallbladder on the ultrasound image Ib. Then, the measurement unit 31 determines an optimal measurement point for the detected gallbladder. Specifically, as shown in Fig. 7, the measurement unit 31 determines two longest distance points on the boundary surrounding the gallbladder region on the ultrasound image Ib as measurement points K1 and K2, and measures the length of a line K3 connecting the measurement points K1 and K2 to each other. In Fig. 7, the length of the line K3 is 56 mm. The measurement unit 31 displays the measurement points K1 and K2 and the line K3 and the measurement target and the length as a measurement result R, that is, gallbladder: 56 mm, on the image display unit 14 through the image processing and storage unit 26 and the display controller 27.

In the present embodiment, the measurement unit 31 determines the position of the detection range for detecting the measurement target based on the position received by the position designation receiving unit 30. However, the present invention is not limited thereto. The measurement unit 31 may further determine a measurement position for measuring the measurement target based on the position received by the position designation receiving unit 30, that is, the positions of the measurement points K1 and K2. For example, in a case where the measurement target is a long blood vessel or the like, the measurement unit 31 determines which diameter is to be measured based on the received position. For example, the measurement unit 31 can determine the shortest line passing through the received position as the line K3 and determine points at both ends of the line K3 as the measurement points K1 and K2.

In a case where the detection range for detecting the measurement target is the entire ultrasound image Ib, the measurement unit 31 does not need to determine the position of the range for detecting the measurement target. For this reason, the measurement unit 31 may determine only the measurement position for measuring the measurement target, that is, the positions of the measurement points K1 and K2.

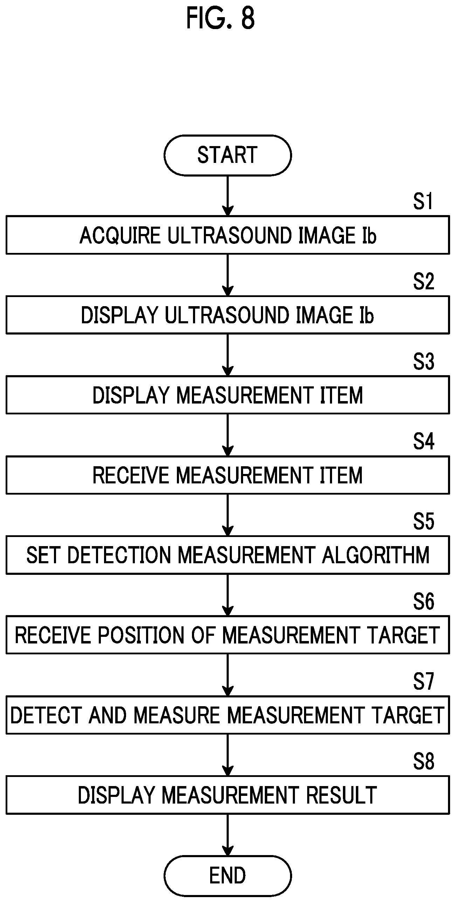

Next, an operation method of an acoustic wave diagnostic apparatus, in which the acoustic wave diagnostic apparatus 1 measures a measurement target in the ultrasound image Ib displayed on the image display unit 14, in the acoustic wave image capturing apparatus 10 described above will be described. Fig. 8 is a flowchart showing a method of a series of processes of the acoustic wave diagnostic apparatus 1 shown in Fig. 2.

As shown in Fig. 8, in the acoustic wave diagnostic apparatus 1, the image processing and storage unit 26 receives and stores image data of the ultrasound image Ib generated by the ultrasound image generation unit 25, thereby acquiring the ultrasound image Ib (step S1). In the present embodiment, the image processing and storage unit 26 receives and stores the image data of the ultrasound image Ib generated by the ultrasound image generation unit 25, thereby acquiring the ultrasound image Ib. However, the present invention is not limited thereto. For example, the ultrasound image Ib may be acquired by inputting the image data of the ultrasound image Ib, which is stored in external storage means in advance, through an input and output unit (not shown) provided in the acoustic wave diagnostic apparatus 1. The present invention can also be applied to a case where the image data of the ultrasound image Ib generated by the ultrasound image generation unit 25 is displayed on the image display unit 14 by the display controller 27 without being temporarily stored in the image processing and storage unit 26.

Then, the display controller 27 displays the image data of the ultrasound image Ib, which is stored in the image processing and storage unit 26 and has been subjected to various kinds of image processing, on the image display unit 14 (step S2). Then, in a case where the measurement function is started by the operator on the UI application, the display controller 27 displays the list N of measurement items relevant to the measurement target on the image display unit 14 as shown in Fig. 4 (step S3).

In a case where the list N of measurement items is displayed on the image display unit 14, the operator designates the gallbladder size N2 as a desired measurement item from the list N of measurement items using the finger P, and the measurement item designation receiving unit 28 receives the gallbladder size N2 selected by the operator as a measurement item (step S4).

Then, the detection measurement algorithm setting unit 29 sets the detection measurement algorithm for the measurement item received by the measurement item designation receiving unit 28, that is, the gallbladder size (step S5).

The position designation receiving unit 30 receives designation of the position of the measurement target, that is, the gallbladder on the ultrasound image Ib displayed on the image display unit 14 (step S6). Then, the measurement unit 31 detects the inside of the detection range based on the detection algorithm set by the detection measurement algorithm setting unit 29, and detects the accurate position and region of the gallbladder on the ultrasound image Ib. The measurement unit 31 determines the optimal measurement points K1 and K2 for the detected gallbladder, and measures the length of the line K3 connecting the measurement points K1 and K2 to each other (step S7). Then, the measurement unit 31 displays the measurement points K1 and K2 and the line K3 and the measurement target and the length as the measurement result R, that is, gallbladder: 56 mm, on the image display unit 14 through the image processing and storage unit 26 and the display controller 27 (step S8). As described above, the acoustic wave diagnostic apparatus 1 measures the measurement target.

According to the acoustic wave diagnostic apparatus 1 and the operation method of the acoustic wave diagnostic apparatus 1 of the present embodiment, the measurement unit 31 can automatically detect and measure a measurement target M1 simply by designating the approximate position of the measurement target M1 on the acoustic wave image displayed on the image display unit 14 by the operator. Therefore, it is possible to measure the measurement target M1 quickly and easily by reducing the time or effort required for the operator to input measurement points and to perform measurement with reduced variations by the operator.

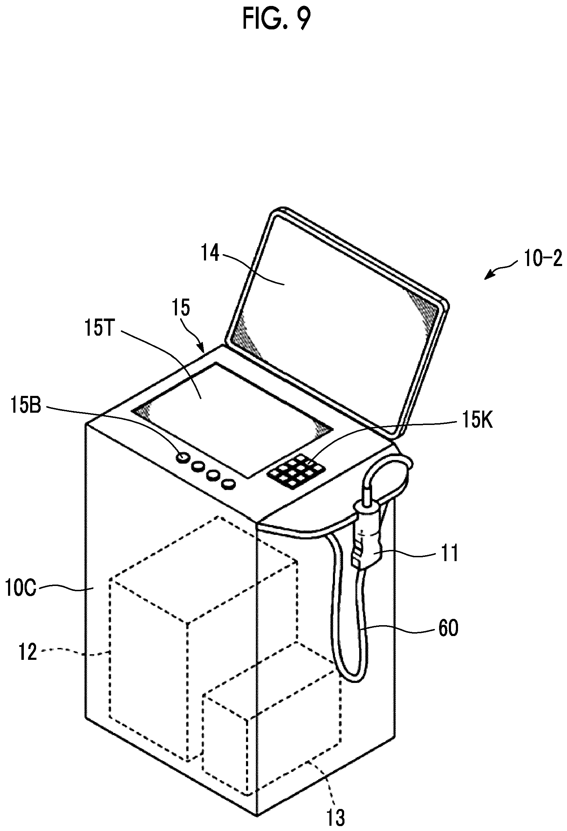

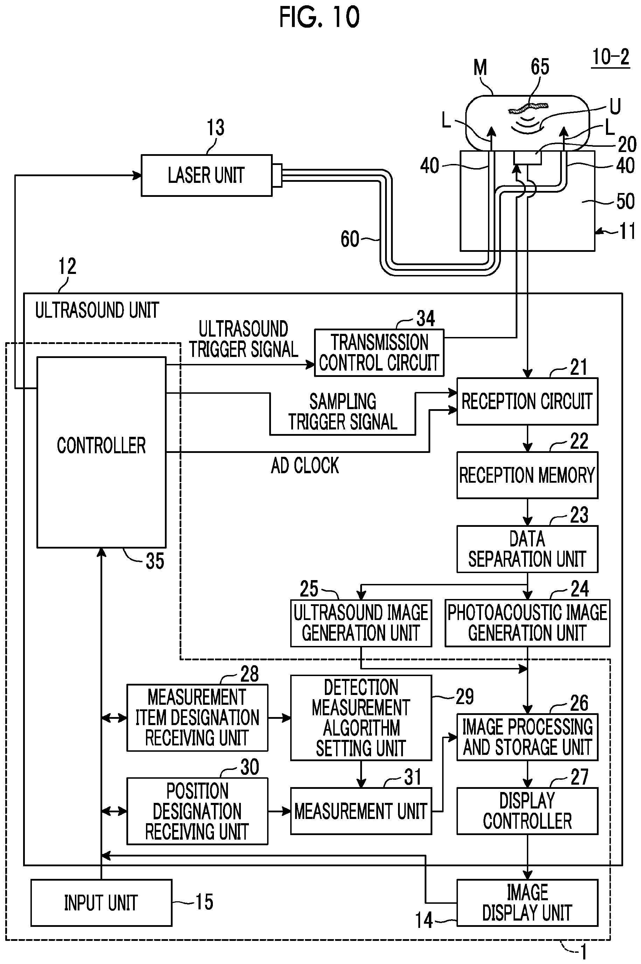

Hereinafter, an acoustic wave image capturing apparatus 10-2 according to a second embodiment of the present invention will be described in detail with reference to the diagrams. Fig. 9 is a perspective view showing the acoustic wave image capturing apparatus 10-2 comprising the acoustic wave diagnostic apparatus 1, and Fig. 10 is a schematic diagram showing the overall configuration of the acoustic wave image capturing apparatus 10-2 shown in Fig. 9.

As an example, the acoustic wave image capturing apparatus 10-2 according to the present embodiment has both a function of generating a photoacoustic image based on a photoacoustic signal and a function of generating an ultrasound image based on a reflected ultrasound wave detection signal. The acoustic wave diagnostic apparatus according to the embodiment of the present invention may be mounted in an apparatus having only the function of generating a photoacoustic image, or may be mounted in an apparatus having only the function of generating an ultrasound image. The acoustic wave diagnostic apparatus according to the embodiment of the present invention can be mounted in any apparatus that can receive or store image data of at least one of a photoacoustic image or an ultrasound image of a two-dimensional image and display the received or stored image data even though the apparatus does not have any of the above-described functions.

As an example, the acoustic wave image capturing apparatus 10-2 is configured as a so-called tower type apparatus, as shown in Fig. 9. As shown in Figs. 9 and 10, the acoustic wave image capturing apparatus 10-2 comprises a probe 11 that is an ultrasound probe, an ultrasound unit 12, a laser unit 13, an image display unit 14, and an input unit 15. The ultrasound unit 12 and the laser unit 13 are housed in a housing 10C. More specifically, the image display unit 14 is an image display screen including, for example, a liquid crystal display device, and is configured by a touch panel to which direct input can be performed by the operator. The input unit 15 also comprises, for example, a touch panel 15T different from the image display unit 14, a plurality of input buttons 15B, a plurality of input keys 15K, and the like. The image display unit 14 of the present embodiment also functions as the input unit 15. Each of the image display unit 14 and the touch panel 15T is configured to be able to display a color image. Hereinafter, the components of the acoustic wave image capturing apparatus 10-2 will be sequentially described.

The probe 11 has a function of emitting measurement light and ultrasound waves toward the subject M that is a living body, for example. In Fig. 10, the shape of the probe 11 is schematically shown. The probe 11 has a function of detecting the acoustic wave U propagating through the subject M. The probe 11 emits measurement light to the subject M, and detects photoacoustic waves generated in the subject due to the emission of the measurement light. In addition, the probe 11 emits (transmits) ultrasound waves (acoustic waves) to the subject M, and detects (receives) reflected ultrasound waves (reflected acoustic waves) that are returned by reflection from the subject M.

In this specification, the "acoustic wave" includes an ultrasound wave or a photoacoustic wave, and the "ultrasound wave" means an elastic wave transmitted by the probe and its reflected wave (reflected ultrasound wave). The "photoacoustic wave" means an elastic wave that is generated in a case where an absorber 65 absorbs measurement light. The acoustic wave emitted from the probe 11 is not limited to the ultrasound wave, and an acoustic wave having an audible frequency may be used as long as an appropriate frequency can be selected according to an examination target, measurement conditions, and the like. As the absorber 65 in the subject M, for example, a blood vessel, a metal member, and the like can be mentioned.

The probe 11 is configured as, for example, a sector scanning probe, a linear scanning probe, or a convex scanning probe. The type of probe used for acquisition of an acoustic wave image is appropriately selected according to an imaging part and the like. An optical fiber 60 that makes a connection between the laser unit 13 and the probe 11 is connected to the probe 11. Laser light L that is measurement light emitted from the laser unit 13 is guided to a light emitting unit 40 by the optical fiber 60.

The probe 11 has the transducer array 20, which is an acoustic wave detector, and the light emitting unit 40. In the example shown in Fig. 10, the probe 11 has two light emitting units 40. The two light emitting units 40 are disposed at positions facing each other with the transducer array 20 interposed therebetween. The transducer array 20 and the two light emitting units 40 are housed in the housing 50.

In the present embodiment, the transducer array 20 also functions as an ultrasound wave transmission element. The transducer array 20 is connected to a circuit for transmitting ultrasound waves, a circuit for receiving acoustic waves, and the like through wiring lines (not shown).

The transducer array 20 has a plurality of ultrasound transducers arranged to be parallel in one direction. The ultrasound transducer is an electroacoustic transducer. The ultrasound transducer is a piezoelectric element formed of, for example, piezoelectric ceramics. Alternatively, the ultrasound transducer may be a piezoelectric element formed of a polymer film, such as polyvinylidene difluoride (PVDF). The ultrasound transducer converts the received acoustic wave U into an electrical signal. Although not shown in Fig. 10, the probe 11 may have an acoustic lens on the subject side of the transducer array 20.

Although an example of the transducer array 20 in which a plurality of ultrasound transducers are arranged in a one-dimensional manner has been described above, the transducer array 20 is not limited thereto. In the transducer array 20, a plurality of ultrasound transducers may be arranged in a two-dimensional manner.

In the transducer array 20, in a case where an alternating voltage is applied to the ultrasound transducer, the ultrasound transducer generates an ultrasound wave having a frequency corresponding to the frequency of the alternating voltage, and the ultrasound wave is transmitted from the transducer array 20. In addition, transmission and reception of ultrasound waves may be separated from each other. That is, for example, ultrasound waves may be transmitted from a position different from the probe 11, and reflected ultrasound waves of the transmitted ultrasound waves may be received by the probe 11.

The light emitting unit 40 emits the laser light L guided by the optical fiber 60 toward the subject M. In the present embodiment, the light emitting unit 40 is configured by the distal end portion of the optical fiber 60, that is, an end portion far from the laser unit 13 that is a light source of measurement light. As shown in Fig. 10, in the present embodiment, the two light emitting units 40 are disposed on both sides of the transducer array 20, for example, in the elevation direction with the transducer array 20 interposed therebetween. The "elevation direction" is a direction perpendicular to the arrangement direction and parallel to the detection surface of the transducer array 20 in a case where a plurality of ultrasound transducers are arranged in a one dimensional manner.

The light emitting unit 40 may include a light guide plate and a diffusion plate that are optically coupled to the distal end of the optical fiber 60. As the light guide plate, for example, an acrylic plate or a quartz plate is used. As the diffusion plate, a lens diffusion plate in which microlenses are randomly disposed on the substrate can be used. As the lens diffusion plate, a holographic diffusion plate may be used, or an engineering diffusion plate may be used. Instead of using the lens diffusion plate, for example, a quartz plate in which diffusion fine particles are dispersed may be used as the diffusion plate.

The laser unit 13 emits the laser light L as measurement light. The laser unit 13 includes a flash lamp excitation Q switch solid-state laser, such as a Q switch alexandrite laser, for example. The laser unit 13 receives a trigger signal emitted from the controller 35 of the ultrasound unit 12, for example. The laser unit 13 outputs the laser light L in a case where a trigger signal is received. It is preferable that the laser unit 13 outputs the pulsed laser light L having a pulse width of 1 to 100 nsec (nanosecond).

The wavelength of the laser light L is appropriately selected according to the light absorption characteristics of the absorber 65 in the subject M that is a measurement target. For example, in a case where the measurement target is hemoglobin in a living body, that is, in the case of imaging a blood vessel, it is preferable that the wavelength of the laser light L is a wavelength belonging to the near-infrared wavelength band. The near-infrared wavelength band means a wavelength band of about 700 to 2500 nm. The wavelength of the laser light L is arbitrary, and is not limited to the wavelength belonging to the near-infrared wavelength band. The laser unit 13 may emit the laser light L having a single wavelength, or may emit the laser light L having a plurality of wavelengths. As a plurality of wavelengths, for example, a combination of 750 nm and 800 nm can be considered. In a case where the laser light L includes a plurality of wavelengths, light beams having the plurality of wavelengths may be simultaneously emitted or may be emitted while being switched alternately.

The laser unit 13 is not limited to including an alexandrite laser. Examples of the laser unit 13 include an yttrium aluminum garnet (YAG)-second harmonic generation (SHG)-optical parametric oscillation (OPO) laser capable of outputting laser light in a near-infrared wavelength band, a YAG laser that does not perform wavelength conversion, or a titanium-sapphire (Ti-Sapphire) laser. The laser unit 13 is not limited to the flash lamp excitation solid-state laser, and may include a laser diode excitation solid-state laser.

The optical fiber 60 guides the laser light L, which is emitted from the laser unit 13, to the two light emitting units 40. Although not particularly limited, for example, a known optical fiber such as a quartz fiber can be used as the optical fiber 60. As the optical fiber 60, for example, one thick optical fiber may be used, or a bundle fiber in which a plurality of optical fiber strands are bundled may be used. In a case where a bundle fiber is used as the optical fiber 60, the laser light L may be incident on the light incidence end surfaces of the plurality of optical fiber strands bundled together, the plurality of optical fiber strands may be branched into two groups, and each of the light emitting ends of the optical fiber strands branched into two groups may be used as the light emitting unit 40.

The ultrasound unit 12 has a reception circuit 21, a reception memory 22, data separation unit 23, a photoacoustic image generation unit 24, an ultrasound image generation unit 25, an image processing and storage unit 26, a display controller 27, a measurement item designation receiving unit 28, a detection measurement algorithm setting unit 29, a position designation receiving unit 30, a measurement unit 31, a transmission control circuit 34, and a controller 35. In the present embodiment, the acoustic wave diagnostic apparatus 1 that is an embodiment of the present invention is configured by the image display unit 14, the input unit 15, the image processing and storage unit 26, the display controller 27, the measurement item designation receiving unit 28, the detection measurement algorithm setting unit 29, the position designation receiving unit 30, the measurement unit 31, and the controller 35.

The controller 35 controls each unit of the acoustic wave diagnostic apparatus 1, and controls each unit of the acoustic wave image capturing apparatus 10-2. The controller 35 comprises a trigger control circuit (not shown). For example, in the case of acquiring a photoacoustic image, the trigger control circuit transmits a light trigger signal to the laser unit 13. In a case where the optical trigger signal is received, the laser unit 13 turns on the flash lamp of the excitation source in the Q switch solid-state laser to start excitation of the laser rod. While the excitation state of the laser rod is maintained, the laser unit 13 can output the laser light L.

The trigger control circuit of the controller 35 transmits a Q switch trigger signal to the laser unit 13 after transmitting the optical trigger signal. In a case where the Q switch trigger signal is received, the laser unit 13 opens the Q switch of the Q switch solid-state laser to output the laser light L. The timing at which the laser light L is output from the laser unit 13 is controlled by the Q switch trigger signal. The controller 35 transmits a Q switch trigger signal, and transmits a sampling trigger signal to the reception circuit 21. The sampling trigger signal defines the start timing of photoacoustic signal sampling in an analog to digital converter (AD converter) of the reception circuit 21. By starting sampling by the reception circuit that receives the sampling trigger signal, the photoacoustic signal can be sampled in synchronization with the output of the laser light L.

In the case of acquiring an ultrasound image, the controller 35 transmits an ultrasound trigger signal for instructing the transmission control circuit 34 to transmit ultrasound waves. In a case where the ultrasound trigger signal is received, the transmission control circuit 34 causes the probe 11 to transmit ultrasound waves. The controller 35 transmits a sampling trigger signal to the reception circuit 21 according to the timing of ultrasound wave transmission, thereby starting the sampling of the reflected ultrasound wave signal.

The reception circuit 21 receives the photoacoustic wave detection signal output from the transducer array 20 of the probe 11, and stores the received detection signal in the reception memory 22. Typically, the reception circuit 21 is configured to include a low noise amplifier, a variable gain amplifier, a low pass filter, and an AD converter. The photoacoustic wave detection signal output from the probe 11 is amplified by the low noise amplifier and then the gain is adjusted according to the depth by the variable gain amplifier, and high-frequency components are cut by the low pass filter and then conversion into a digital signal is performed by the AD converter and the digital signal is stored in the reception memory 22. The reception circuit 21 is configured by, for example, one integrated circuit (IC). The above-described low pass filter is provided to prevent aliasing noise from occurring at the time of AD conversion. The cut-off frequency of the low pass filter is generally set to a frequency that is about half the sampling frequency of AD conversion. Specifically, the cut-off frequency of the low pass filter is set to about 10 MHz to 30 MHz.

In the present embodiment, the probe 11 outputs a photoacoustic wave detection signal and a reflected ultrasound wave detection signal. A photoacoustic wave detection signal and a reflected ultrasound wave detection signal that are digitized are stored in the reception memory 22. More specifically, the photoacoustic wave detection signal is a signal obtained by detecting a photoacoustic wave that is generated from the inside of the subject M by receiving the laser light L emitted toward the subject M. On the other hand, the reflected ultrasound wave detection signal is a signal obtained by detecting a reflected acoustic wave that is a reflected wave of an ultrasound wave, which is an acoustic wave emitted toward the subject M, in the subject M. The data separation unit 23 reads the photoacoustic image data, that is, the digitized photoacoustic wave detection signal from the reception memory 22, and transmits the digitized photoacoustic wave detection signal to the photoacoustic image generation unit 24. In addition, the data separation unit 23 reads the reflected ultrasound image data, that is, the digitized reflected ultrasound wave detection signal from the reception memory 22, and transmits the digitized reflected ultrasound wave detection signal to the ultrasound image generation unit 25.

The photoacoustic image generation unit 24 generates a photoacoustic image by reconstructing the photoacoustic wave detection signal received from the reception memory 22 through the data separation unit 23. Specifically, the photoacoustic image generation unit 24 adds a photoacoustic wave detection signal based on a signal output from each ultrasound transducer with a delay time corresponding to the position of each ultrasound transducer of the transducer array 20, thereby generating a photoacoustic wave detection signal for one line (delay addition method). The photoacoustic image generation unit 24 may perform reconstruction using a circular back projection (CBP) method instead of the delay addition method. Alternatively, the photoacoustic image generation unit 24 may perform reconstruction using a Hough transform method or a Fourier transform method. The reconstructed photoacoustic wave detection signals for a plurality of lines are subjected to signal processing, such as detection processing and logarithmic conversion processing, and then are transmitted to the display controller 27 through the image processing and storage unit 26 as signals for displaying a photoacoustic image (tomographic image) regarding a cross section of the subject M.

The ultrasound image generation unit 25 performs basically the same processing as for the photoacoustic wave detection signal on the reflected ultrasound wave detection signal received from the reception memory 22 through the data separation unit 23, thereby generating reflected ultrasound wave detection signals for a plurality of lines indicating an ultrasound image (tomographic image). The reflected ultrasound wave detection signal generated as described above is transmitted to the display controller 27 through the image processing and storage unit 26 as a signal for displaying an ultrasound image regarding a cross section of the subject M.

The image processing and storage unit 26 performs various kinds of processing for image quality improvement, such as brightness correction, gradation correction, sharpness correction, and color correction, on the image data of the photoacoustic image and the ultrasound image. The image processing and storage unit 26 of the present embodiment also functions as a storage unit that stores various databases and a storage unit that stores, as image data, a signal for displaying the image generated by the photoacoustic image generation unit 24 or the ultrasound image generation unit 25. Although the image processing and storage unit 26 of the acoustic wave diagnostic apparatus 1 and the acoustic wave image capturing apparatus 10-2 of the present embodiment comprises both an image processing function and a function as a storage unit, the acoustic wave diagnostic apparatus according to the embodiment of the present invention may have only a function as a storage unit without having an image processing function.

The display controller 27 displays a photoacoustic image on the image display unit 14 based on the above-described signal for displaying the photoacoustic image. In addition, based on the above-described signal for displaying the ultrasound image, the ultrasound image Ib is displayed on the image display unit 14 as shown in Fig. 3. These two types of images are displayed on the image display unit 14 separately or as a composite image. In the latter case, the display controller 27 performs image composition by superimposing a photoacoustic image and an ultrasound image, for example. In a case where the photoacoustic image and the ultrasound image are superimposed and displayed, a portion that cannot be imaged in the photoacoustic image can be observed in the ultrasound image. In the acoustic wave diagnostic apparatus 1 of the present embodiment, the acoustic wave image displayed on the image display unit 14 will be described below as the ultrasound image Ib. However, the present invention is not limited thereto, and a photoacoustic wave image or a composite image in which a photoacoustic image and an ultrasound image are superimposed can be displayed and measured.

The measurement item designation receiving unit 28 receives designation of a measurement item relevant to the measurement target. Specifically, in a case where the measurement function is started on a user interface (UI) application by the operator, the list N of measurement items relevant to the measurement target is displayed on the image display unit 14 as shown in Fig. 4. In each measurement item displayed in the list N of measurement items, the name of the organ and the measurement content regarding the organ are described. For example, an abdominal aorta diameter N1, a gallbladder size N2, a kidney size N3, a gallbladder wall thickness, and the like are displayed. Although the name of the organ and the measurement content regarding the organ are displayed as measurement items relevant to the measurement target in the present embodiment, the present invention is not limited thereto, and only the name of the organ may be displayed. In addition, without being limited to the name of the organ, only items relevant to the names or abnormalities of lesions such as tumors, cysts, and bleeding may be displayed, or the names of lesions and the measurement content regarding the lesions may be displayed, or an item regarding abnormality and the measurement content regarding the item regarding abnormality may be displayed. The display content can be appropriately changed. For example, in the case of displaying only the name of an organ, the displayed name of the organ is associated with the measurement content, such as measuring the diameter, measuring the size, or measuring the length. Specifically, an association table is stored in the image processing and storage unit 26, and the measurement content is selected based on the table in a case where the name of an organ is designated.

In a case where the list N of measurement items is displayed on the image display unit 14 as shown in Fig. 4, the operator selects, for example, the gallbladder size N2 as a desired measurement item from the list N of measurement items using the finger P as shown in Fig. 5. The measurement item designation receiving unit 28 receives the gallbladder size N2 selected by the operator as a measurement item. Although the measurement item designation receiving unit 28 of the present embodiment receives a selection item selected by a touch panel, the present invention is not limited thereto. For example, an input device, such as a mouse, a track pad, or a track ball, may be used as an input unit, and a selection item selected by the input unit may be received.

The detection measurement algorithm setting unit 29 sets a detection algorithm and a measurement algorithm based on the measurement item received by the measurement item designation receiving unit 28. In general, an algorithm for detecting a measurement target on an image differs depending on the type of organ, lesion, or the like. In addition, the algorithm for measuring a measurement target on the image also differs depending on the measurement content, such as measuring the diameter, measuring the size, or measuring the length. The detection measurement algorithm setting unit 29 stores an algorithm corresponding to each measurement target and an algorithm corresponding to each measurement content as an association table, and sets a detection measurement algorithm with reference to the above-described association table in a case where the measurement item designation receiving unit 28 receives a measurement item. As the detection measurement algorithm, a known algorithm that is generally used can be used. The algorithm referred to herein defines a procedure (calculation procedure) for achieving a certain purpose (detection or measurement). For example, the algorithm referred to herein is implemented as a software program in an apparatus and is executed by a processor.

For example, for a measurement target detection algorithm, there is a method in which typical pattern data is stored in advance as a template, a pattern data similarity is calculated while searching for an image with a template, and it is considered that a target is present in a place where the similarity is equal to or greater than a predetermined threshold value and is the maximum. For the calculation of the similarity, in addition to simple template matching, for example, a machine learning method described in Csurka et al.: Visual Categorization with Bags of Keypoints, Proc. of ECCV Workshop on Statistical Learning in Computer Vision, pp. 59-74 (2004) or a general image recognition method using deep learning described in Krizhevsk et al.: ImageNet Classification with Deep Convolutional Neural Networks, Advances in Neural Information Processing Systems 25, pp.1106-1114 (2012) can be used.

The position designation receiving unit 30 receives designation of the position of a measurement target on the ultrasound image Ib displayed on the image display unit 14. Specifically, in a case where the measurement item received by the measurement item designation receiving unit 28 is the gallbladder size N2, the operator desires to measure the size of the gallbladder. Therefore, the measurement target is the gallbladder. Then, as shown in Fig. 6, the operator designates a position where the operator himself or herself thinks that the gallbladder is present on the ultrasound image Ib, for example, the tip of the arrow in Fig. 6 using the finger P. The position designation receiving unit 30 receives the position designated by the operator as a measurement target position.

In a case where the designation of the position of the measurement target is received by the position designation receiving unit 30, the measurement unit 31 detects the measurement target based on the received position and the detection measurement algorithm set by the detection measurement algorithm setting unit 29, and performs measurement for the detected measurement target. The measurement unit 31 determines the position of a detection range for detecting the measurement target based on the received position, and detects the inside of the detection range at the determined position. The size of the detection range is set in the measurement unit 31 in advance, but can be changed by the operator.

Specifically, in a case where the measurement item received by the measurement item designation receiving unit 28 is the gallbladder size N2, the measurement unit 31 first detects the gallbladder on the ultrasound image Ib within the detection range based on the detection algorithm set by the detection measurement algorithm setting unit 29. The measurement unit 31 detects the accurate position and region of the gallbladder on the ultrasound image Ib. Then, the measurement unit 31 determines an optimal measurement point for the detected gallbladder. Specifically, as shown in Fig. 7, the measurement unit 31 determines two longest distance points on the boundary surrounding the gallbladder region on the ultrasound image Ib as measurement points K1 and K2, and measures the length of a line K3 connecting the measurement points K1 and K2 to each other. In Fig. 7, the length of the line K3 is 56 mm. The measurement unit 31 displays the measurement points K1 and K2 and the line K3 and the measurement target and the length as a measurement result R, that is, gallbladder: 56 mm, on the image display unit 14 through the image processing and storage unit 26 and the display controller 27.

In the present embodiment, the measurement unit 31 determines the position of the detection range for detecting the measurement target based on the position received by the position designation receiving unit 30. However, the present invention is not limited thereto, and the measurement unit 31 may further determine the measurement position for measuring the measurement target, that is, the positions of the measurement points K1 and K2. For example, in a case where the measurement target is a long blood vessel or the like, the measurement unit 31 determines which diameter is to be measured based on the received position. For example, the measurement unit 31 can determine the shortest line passing through the received position as the line K3 and determine points at both ends of the line K3 as the measurement points K1 and K2.

In a case where the detection range for detecting the measurement target is the entire ultrasound image Ib, the measurement unit 31 does not need to determine the position of the range for detecting the measurement target. For this reason, the measurement unit 31 may determine only the measurement position for measuring the measurement target, that is, the positions of the measurement points K1 and K2.

Next, an operation method of an acoustic wave diagnostic apparatus, in which the acoustic wave diagnostic apparatus 1 measures a measurement target in the ultrasound image Ib displayed on the image display unit 14, in the acoustic wave image capturing apparatus 10-2 described above will be described.

As shown in Fig. 8, in the acoustic wave diagnostic apparatus 1, the image processing and storage unit 26 receives and stores image data of the ultrasound image Ib generated by the ultrasound image generation unit 25, thereby acquiring the ultrasound image Ib (step S1). In the present embodiment, the image processing and storage unit 26 receives and stores the image data of the ultrasound image Ib generated by the ultrasound image generation unit 25, thereby acquiring the ultrasound image Ib. However, the present invention is not limited thereto. For example, the ultrasound image Ib may be acquired by inputting the image data of the ultrasound image Ib, which is stored in external storage means in advance, through an input and output unit (not shown) provided in the acoustic wave diagnostic apparatus 1. The present invention can also be applied to a case where the image data of the ultrasound image Ib generated by the ultrasound image generation unit 25 is displayed on the image display unit 14 by the display controller 27 without being temporarily stored in the image processing and storage unit 26.

Then, the display controller 27 displays the image data of the ultrasound image Ib, which is stored in the image processing and storage unit 26 and has been subjected to various kinds of image processing, on the image display unit 14 (step S2). Then, in a case where the measurement function is started by the operator on the UI application, the display controller 27 displays the list N of measurement items relevant to the measurement target on the image display unit 14 as shown in Fig. 4 (step S3).

In a case where the list N of measurement items is displayed on the image display unit 14, as shown in Fig. 5, the operator designates the gallbladder size N2 as a desired measurement item from the list N of measurement items using the finger P, and the measurement item designation receiving unit 28 receives the gallbladder size N2 selected by the operator as a measurement item (step S4).

Then, the detection measurement algorithm setting unit 29 sets the detection measurement algorithm for the measurement item received by the measurement item designation receiving unit 28, that is, the gallbladder size (step S5).

The position designation receiving unit 30 receives designation of the position of the measurement target, that is, the gallbladder on the ultrasound image Ib displayed on the image display unit 14 (step S6). Then, the measurement unit 31 detects the inside of the detection range based on the detection algorithm set by the detection measurement algorithm setting unit 29, and detects the accurate position and region of the gallbladder on the ultrasound image Ib. The measurement unit 31 determines the optimal measurement points K1 and K2 for the detected gallbladder, and measures the length of the line K3 connecting the measurement points K1 and K2 to each other (step S7). Then, the measurement unit 31 displays the measurement points K1 and K2 and the line K3 and the measurement target and the length as the measurement result R, that is, gallbladder: 56 mm, on the image display unit 14 through the image processing and storage unit 26 and the display controller 27 (step S8). As described above, the acoustic wave diagnostic apparatus 1 measures the measurement target.

According to the acoustic wave diagnostic apparatus 1 and the operation method of the acoustic wave diagnostic apparatus 1 of the present embodiment, the measurement unit 31 can automatically detect and measure a measurement target M1 simply by designating the approximate position of the measurement target M1 on the acoustic wave image displayed on the image display unit 14 by the operator. Therefore, it is possible to measure the measurement target M1 quickly and easily by reducing the time or effort required for the operator to input measurement points and to perform measurement with reduced variations by the operator.

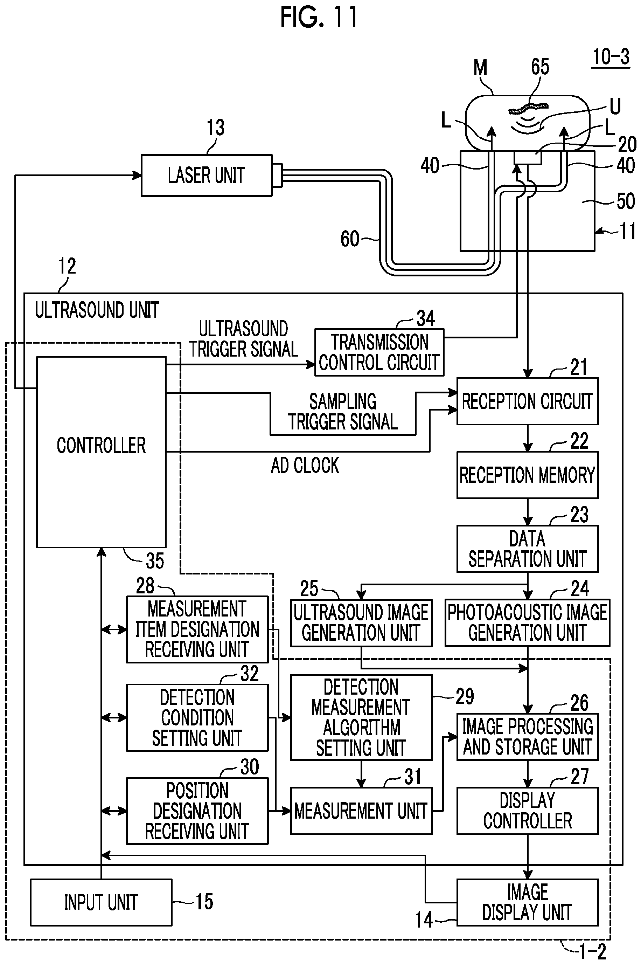

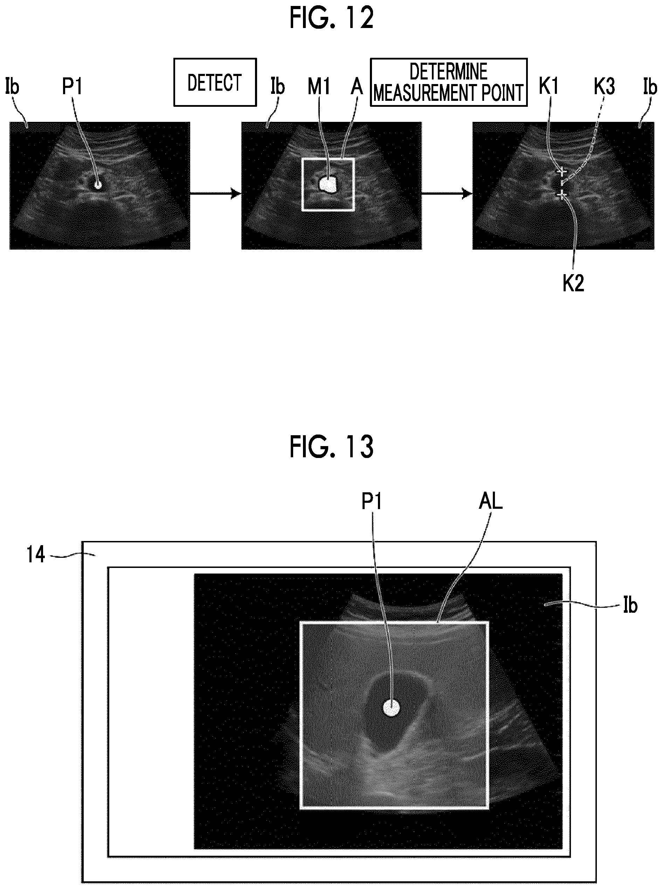

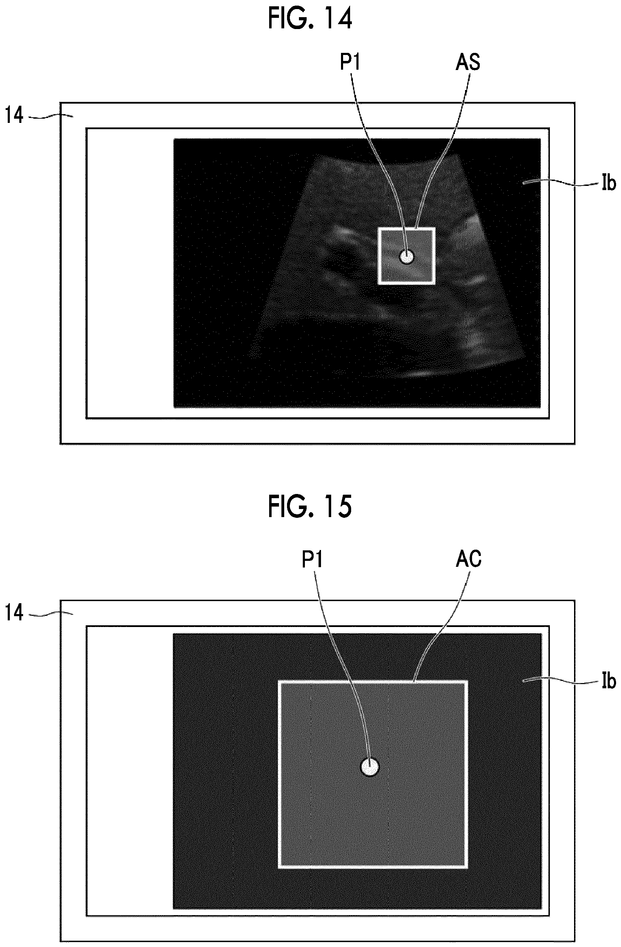

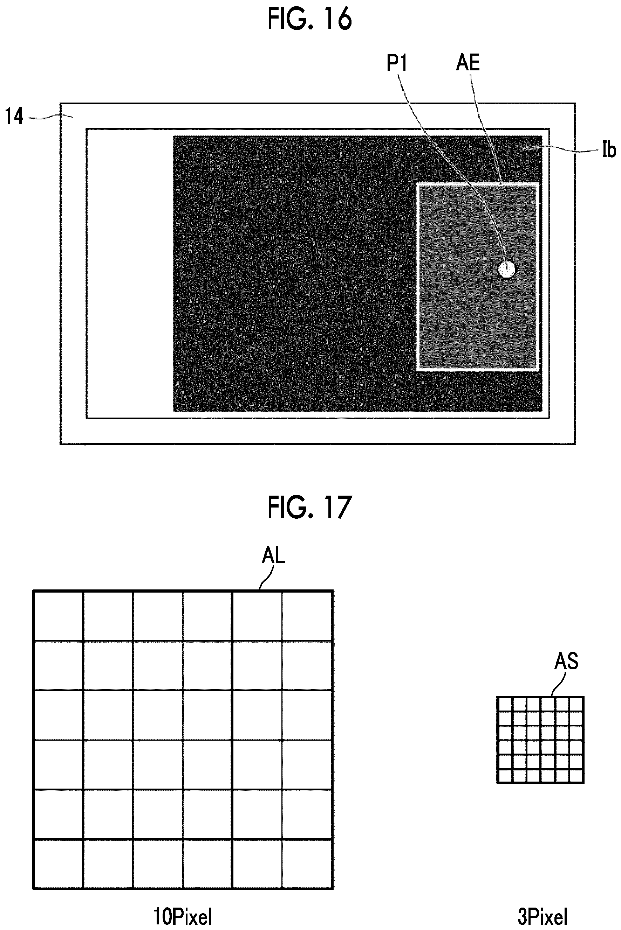

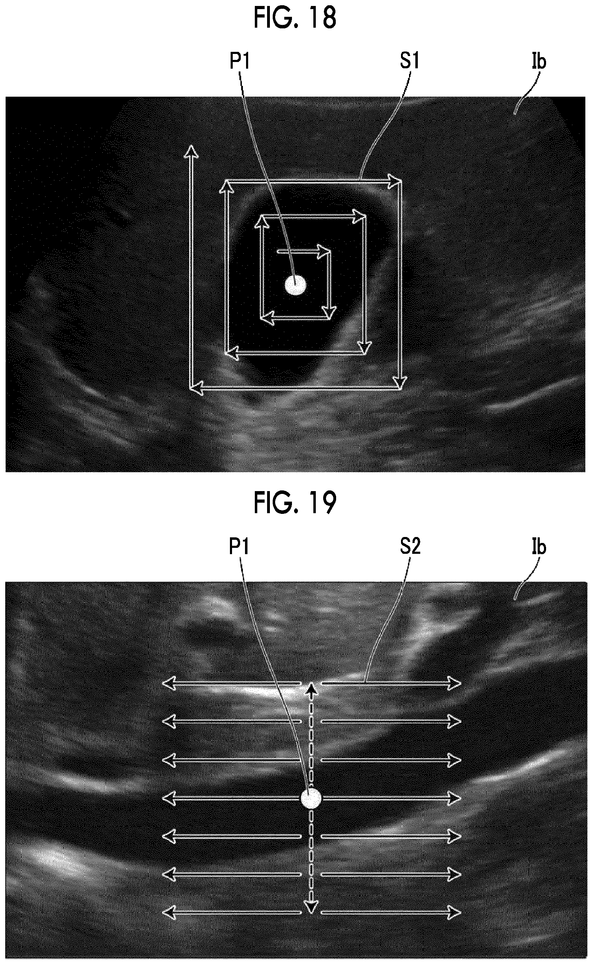

Next, an acoustic wave image capturing apparatus 10-3 comprising an acoustic wave diagnostic apparatus 1-2 according to the second embodiment of the present invention will be described in detail with reference to the diagrams. Fig. 11 is a schematic diagram showing the overall configuration of the acoustic wave image capturing apparatus 10-3 comprising the acoustic wave diagnostic apparatus 1-2 according to the second embodiment of the present invention, Fig. 12 is a diagram illustrating processing by the acoustic wave diagnostic apparatus 1-2 shown in Fig. 11, Fig. 13 is a diagram showing an example of the size of a detection range on the ultrasound image Ib, Fig. 14 is a diagram showing another example of the size of the detection range on the ultrasound image Ib, Fig. 15 is a diagram showing an example of the position of the detection range on the ultrasound image Ib, Fig. 16 is a diagram showing another example of the position of the detection range on the ultrasound image Ib, Fig. 17 is a diagram illustrating the detection accuracy within the detection range, Fig. 18 is a diagram showing an example of the detection order on the ultrasound image Ib, and Fig. 19 is a diagram showing another example of the detection order on the ultrasound image Ib. The acoustic wave diagnostic apparatus 1-2 shown in Fig. 11 is obtained by further providing a detection condition setting unit 32 in the acoustic wave diagnostic apparatus 1 shown in Fig. 9, and the other components are the same as those of the acoustic wave diagnostic apparatus 1 shown in Fig. 9. Therefore, the same components are denoted by the same reference numerals for the sake of convenience, and the description thereof will be omitted herein.

The acoustic wave diagnostic apparatus 1-2 shown in Fig. 11 comprises the detection condition setting unit 32. The detection condition setting unit 32 determines at least one of the position, size, detection accuracy, or detection order of the detection range A of the measurement target M1 based on at least one of a position P1 on the ultrasound image Ib shown in Fig. 12 received by the position designation receiving unit 30 or the measurement item received by the measurement item designation receiving unit 28.

Specifically, the detection condition setting unit 32 determines the size of the detection range A of the measurement target M1 based on the measurement item. For example, in a case where the measurement target M1 is a relatively large organ, such as a kidney, the measurement unit 31 detects, for example, a quadrangular detection range AL of 100 pixels vertically and horizontally with the received position PI as the center, as shown in Fig. 13.

On the other hand, in a case where the measurement target M1 is a relatively small organ, such as the common bile duct or aorta, the measurement unit 31 detects, for example, a quadrangular detection range AS of 20 pixels vertically and horizontally with the received position PI as the center, as shown in Fig. 14. Since the range in which the presence of the measurement target M1 is assumed differs depending on the size of the measurement target M1, the processing time required to detect the measurement target M1 can be shortened by changing the size of the detection range A according to the size of the measurement target M1.

The detection condition setting unit 32 determines the position of the detection range A of the measurement target M1 based on the position P1 on the ultrasound image Ib shown in Fig. 12 received by the position designation receiving unit 30. For example, as shown in Fig. 15, in a case where the position PI is present near the center of the ultrasound image Ib, the measurement unit 31 detects a quadrangular detection range AC that is vertically and horizontally symmetrical with the position PI as the center.

On the other hand, for example, as shown in Fig. 16, in a case where the position PI is present near the right end portion of the ultrasound image Ib, the measurement unit 31 detects a quadrangular detection range AC that is vertically symmetrical and horizontally asymmetrical so as not to protrude from the ultrasound image Ib with the position PI as the center. The range in which the presence of the measurement target M1 is assumed differs depending on the size of the measurement target M1. Therefore, the size of the detection range A may be reduced by changing the position of the detection range A according to the position PI received by the position designation receiving unit 30. In a case where the size of the detection range A is reduced, the processing time required to detect the measurement target M1 can be shortened.

The detection condition setting unit 32 determines the detection accuracy of the detection range A of the measurement target M1 based on the measurement item. For example, in a case where the measurement target M1 is a relatively large organ, such as a kidney, the measurement unit 31 roughly detects, for example, six 10-pixel regions horizontally and six 10-pixel regions vertically in the detection range AL as shown in Fig. 17.

On the other hand, in a case where the measurement target M1 is a relatively small organ, such as the common bile duct or aorta, the measurement unit 31 finely detects, for example, six 3-pixel regions horizontally and six 3-pixel regions vertically in the detection range AS as shown in Fig. 17. Since the range in which the presence of the measurement target M1 is assumed differs depending on the size of the measurement target M1, the balance of the detection accuracy and the processing time required to detect the measurement target M1 and can be improved by changing the detection accuracy in the detection range A according to the size of the measurement target M1. In the present embodiment, in a case where the detection accuracy is changed based on the size of the measurement target M1, the size of the detection range is also changed based on the size of the measurement target M1. However, the present invention is not limited thereto, and only the detection accuracy may be changed.

The detection condition setting unit 32 determines the detection order of the measurement target M1 based on the measurement item and the position PI received by the position designation receiving unit 30. For example, in a case where the measurement item is a round organ, such as a gallbladder short axis surface size or an abdominal aorta short axis diameter, the detection condition setting unit 32 determines the detection order of the measurement target M1 so that the detection is performed in the order indicated by a scanning line S1 in a spiral shape from the center toward the outside with the position PI as the center, as shown in Fig. 18. The interval between the scanning lines S1 may be equal, or the interval may be increased toward the outside or can be appropriately changed. The scanning may be clockwise or counterclockwise, or can be appropriately changed. Although the spiral shape is set in the present embodiment, the present invention is not limited thereto. For example, a plurality of quadrangles whose sizes increase toward the outside with the position PI as the center may be used as scanning lines. In this case, the interval between the scanning lines may be equal, or the interval may be increased toward the outside or can be appropriately changed. The scanning may be clockwise or counterclockwise, or the rotation direction may be changed for each quadrangle or can be appropriately changed. The shape of the scanning line is not limited to a quadrangle, and the shape can be appropriately changed.

On the other hand, in a case where the measurement item is a horizontally long organ, such as the inferior aorta or common bile duct, the detection condition setting unit 32 determines the detection order of the measurement target M1 so that detection in the horizontal direction (arrow S2 in the diagram) is performed and then detection in the vertical direction is performed as shown in Fig. 19. In this case, detection in the horizontal direction passing through the position PI is performed in the order of the left direction and the right direction, and then upward movement is made to perform detection in the horizontal direction in the order of the left direction and the right direction, and downward movement from the position PI is made to perform detection in the horizontal direction in the order of the left direction and the right direction, so that the detection is performed in a direction away from the position PI in the order of the upward direction and the downward direction. The order of detection is not limited thereto, and detection in the horizontal direction on the lower side may be performed after detection in the horizontal direction on the upper side is entirely performed, or the order of detection can be appropriately changed. By determining the detection order according to the shape of the measurement target M1, the speed of finding the measurement target M1 can be increased. In the present embodiment, the detection condition setting unit 32 changes the size or the detection accuracy of the detection range described above, and determines the detection order of the measurement target M1 based on the measurement item and the position PI received by the position designation receiving unit 30. However, the detection condition setting unit 32 of the present invention is not limited thereto. The detection condition setting unit 32 may change only the size or the detection accuracy of the detection range described above, or may determine only the detection order of the measurement target M1.

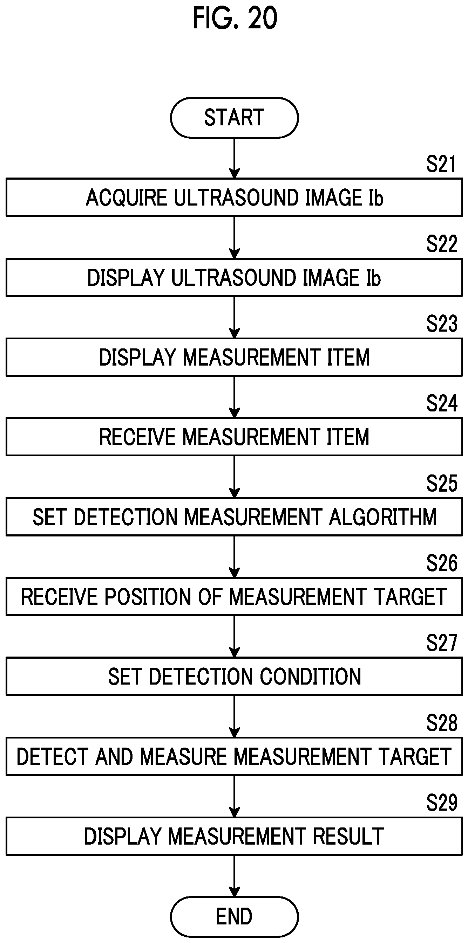

Next, an operation method of an acoustic wave diagnostic apparatus, in which the acoustic wave diagnostic apparatus 1-2 measures a measurement target in the ultrasound image Ib displayed on the image display unit 14, in the acoustic wave image capturing apparatus 10-3 described above will be described. Fig. 20 is a flowchart showing a method of a series of processes of the acoustic wave diagnostic apparatus 1-2 shown in Fig. 11. Since steps S21 to S26 in Fig. 20 are the same as steps S1 to S16 in the flowchart of Fig. 8, the description thereof will be omitted herein.

In the acoustic wave diagnostic apparatus 1-2, as shown in Fig. 20, in a case where the position designation receiving unit 30 receives designation of the position of a measurement target on the ultrasound image Ib displayed on the image display unit 14 (step S26), the detection condition setting unit 32 determines at least one of the position, size, detection accuracy, or detection order of the detection range A of the measurement target M1 as described above based on at least one of the position PI on the ultrasound image Ib received by the position designation receiving unit 30 or the measurement item received by the measurement item designation receiving unit 28 (step S27). Then, the measurement unit 31 detects the inside of the detection range set by the detection condition setting unit 32 based on the detection algorithm set by the detection measurement algorithm setting unit 29, and detects the accurate position and region of the measurement target M1 on the ultrasound image Ib. As shown in Fig. 12, the measurement unit 31 determines the optimal measurement points K1 and K2 for the detected measurement target M1, and measures the length of the line K3 connecting the measurement points K1 and K2 to each other (step S28). Then, the measurement unit 31 displays the measurement points K1 and K2 and the line K3, the measurement target M1 and the size or length of the measurement target M1 as the measurement result R, and the like on the image display unit 14 through the image processing and storage unit 26 and the display controller 27 (step S29). As described above, the acoustic wave diagnostic apparatus 1-2 measures the measurement target.

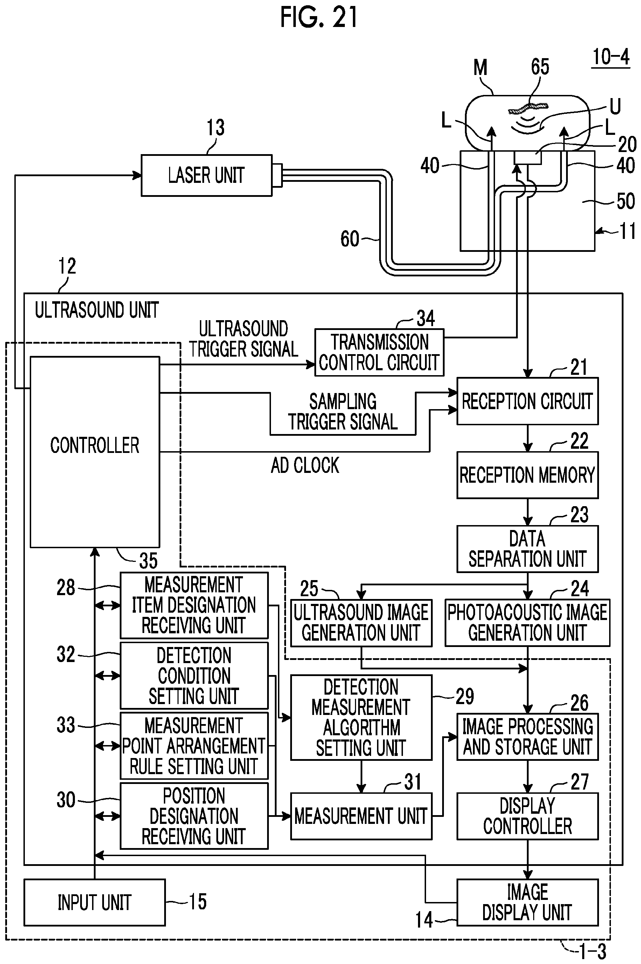

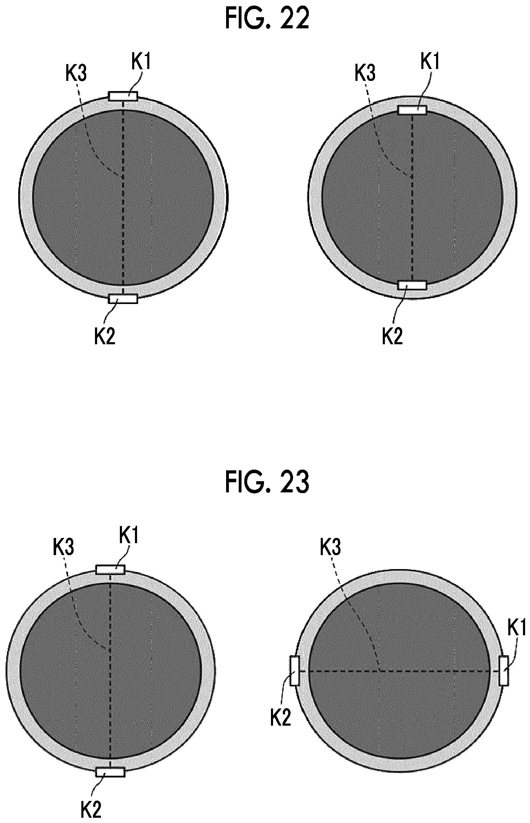

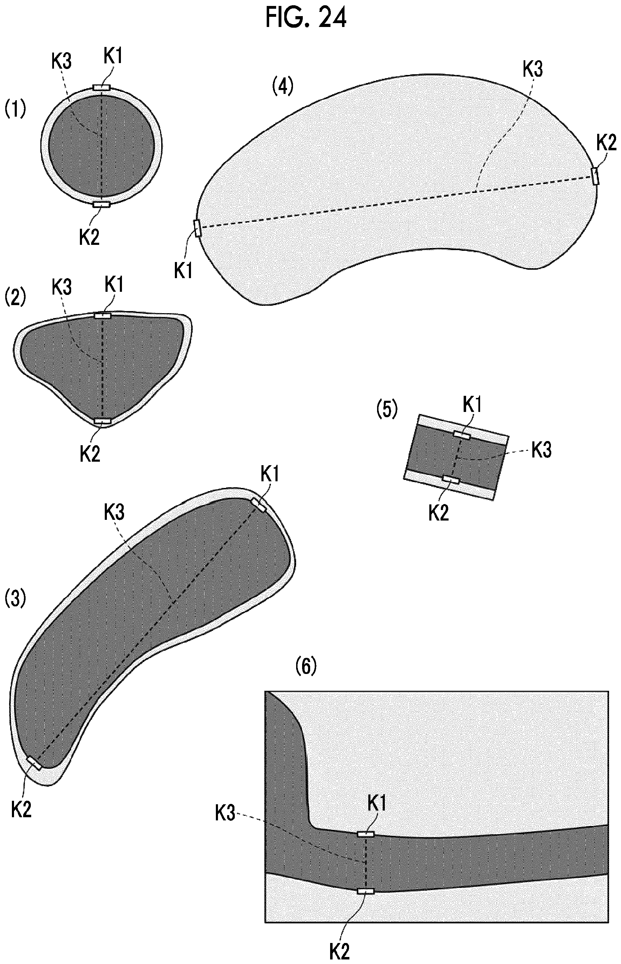

Next, an acoustic wave image capturing apparatus 10-4 comprising an acoustic wave diagnostic apparatus 1-3 according to the third embodiment of the present invention will be described in detail with reference to the diagrams. Fig. 21 is a schematic diagram showing the overall configuration of the acoustic wave image capturing apparatus 10-4 comprising the acoustic wave diagnostic apparatus 1-3 according to the third embodiment of the present invention, Fig. 22 is a diagram showing an example of the arrangement of measurement points, Fig. 23 is a diagram showing another example of the arrangement of measurement points, and Fig. 24 is an example of the arrangement of measurement points in different measurement items. The acoustic wave diagnostic apparatus 1-3 shown in Fig. 21 is obtained by further providing a measurement point arrangement rule setting unit 33 in the acoustic wave diagnostic apparatus 1-2 shown in Fig. 11, and the other components are the same as those of the acoustic wave diagnostic apparatus 1-2 shown in Fig. 11. Therefore, the same components are denoted by the same reference numerals for the sake of convenience, and the description thereof will be omitted herein.

The acoustic wave diagnostic apparatus 1-3 shown in Fig. 21 comprises the measurement point arrangement rule setting unit 33. The measurement point arrangement rule setting unit 33 sets the measurement point arrangement rule for the measurement target M1 based on the measurement item received by the measurement item designation receiving unit 28. The measurement point arrangement rule may differ depending on the measurement item. For example, as shown in the left diagram of Fig. 22, the measurement points K1 and K2 are arranged on the outer sides of a measurement target wall and a distance K3 from the outer side to the outer side of the wall is measured, or as shown in the right diagram of Fig. 22, the measurement points K1 and K2 are arranged on the inner sides of a measurement target wall and a distance K3 from the inner side to the inner side of the wall is measured. In addition, as shown in the left diagram of Fig. 23, the measurement points K1 and K2 are arranged in the vertical direction and a distance K3 in the vertical direction is measured, or as shown in the right diagram of Fig. 23, the measurement points K1 and K2 are arranged in the horizontal direction and a distance K3 in the horizontal direction is measured.

It is preferable that the measurement unit 31 performs measurement for the measurement point set based on the preferable arrangement rule set according to the measurement item. Since the operators have different preferences for the above-described arrangement rule, a plurality of measurement point arrangement rules may be stored in advance in the image processing and storage unit 26, and the operator may select one of the plurality of measurement point arrangement rules. The measurement point arrangement rule can be configured as a table associated with measurement items.

Here, a specific measurement point arrangement rule will be described. Table 1 below is an example of the measurement point arrangement rule associated with measurement items, and includes an association table.

**[Table 1]**

| | Measurement items | Measurement locations | Measurement points |
|---|---|---|---|
| (1) | abdominal aorta | maximum diameter in the vertical direction passing through the center of gravity | from the outer side to the outer side of the wall |
| (2) | short axis size of gallbladder | maximum diameter in the vertical direction passing through the center of gravity | from the inner side to the inner side of the wall |
| (3) | long axis size of gallbladder | two points having a maximum distance therebetween among two points on the boundary | from the inner side to the inner side of the wall |
| (4) | kidney size | two points having a maximum distance therebetween among two points on the boundary | those corresponding to the wall are not visible |
| (5) | common bile duct | inner diameter perpendicular to tube structure | from the inner side to the inner side of the wall |
| (6) | inferior vena cava | inner diameter perpendicular to travel direction | from the inner side to the inner side of the wall |

As shown in Table 1 and Fig. 24, in a case where the measurement item is (1) abdominal aorta, the measurement location is a maximum diameter in the vertical direction passing through the center of gravity. In this case, the measurement points K1 and K2 are arranged on the outer sides of the wall, and the distance K3 from the outer side to the outer side of the wall is measured. In a case where the measurement item is (2) short axis size of gallbladder, the measurement location is a maximum diameter in the vertical direction passing through the center of gravity. In this case, the measurement points K1 and K2 are arranged on the inner sides of the wall, and the distance K3 from the inner side to the inner side of the wall is measured. In a case where the measurement item is (3) long axis size of gallbladder, two points on the boundary of the gallbladder region. In this case, the measurement points K1 and K2 are arranged on the inner sides of the wall, and the distance K3 from the inner side to the inner side of the wall is measured. In a case where the measurement item is (4) kidney size, the measurement locations are two points having a maximum distance therebetween among two points on the boundary of the kidney region. In this case, since those corresponding to the wall are not visible, the measurement points K1 and K2 are arranged at the above two points, and the distance K3 between the measurement points K1 and K2 is measured. In a case where the measurement item is (5) common bile duct, the measurement location is an inner diameter perpendicular to the tube structure. Accordingly, the measurement points K1 and K2 are arranged on the inner sides of the wall, and the distance K3 from the inner side to the inner side of the wall is measured. In a case where the measurement item is (6) inferior vena cava, the measurement location is an inner diameter perpendicular to the travel direction of blood. Accordingly, the measurement points K1 and K2 are arranged on the boundary of the inferior vena cava region, and the distance K3 between the measurement points K1 and K2 is measured. The association table is not limited to the table shown in Table 1, and can be appropriately selected or changed by the operator.

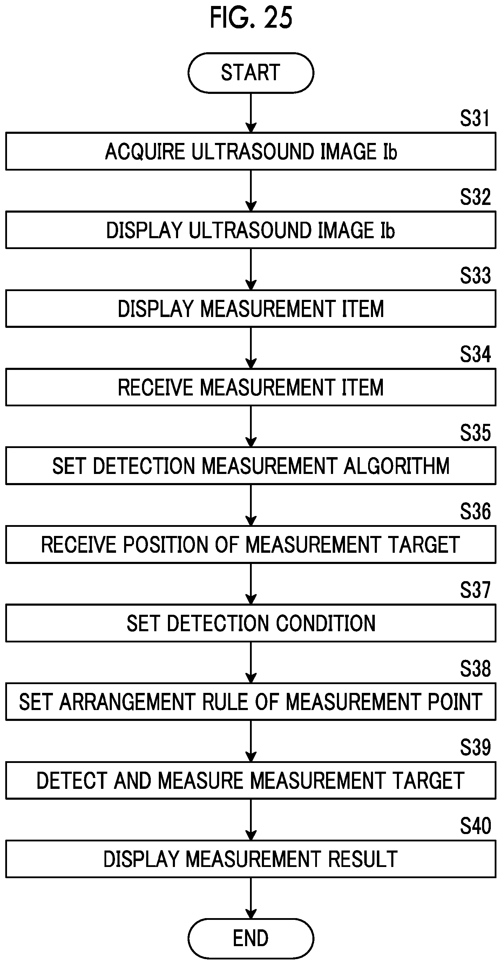

Next, an operation method of the acoustic wave diagnostic apparatus 1-3, in which the acoustic wave diagnostic apparatus 1-3 measures a measurement target in the ultrasound image Ib displayed on the image display unit 14, in the acoustic wave image capturing apparatus 10-4 described above will be described. Fig. 25 is a flowchart showing a method of a series of processes of the acoustic wave diagnostic apparatus 1-3 shown in Fig. 21. Since steps S31 to S37 in Fig. 25 are the same as steps S21 to S27 in the flowchart of Fig. 20, the description thereof will be omitted herein.

In the acoustic wave diagnostic apparatus 1-3, as shown in Fig. 25, the detection condition setting unit 32 determines at least one of the position, size, detection accuracy, or detection order of the detection range A of the measurement target M1 based on at least one of the position P1 on the ultrasound image Ib received by the position designation receiving unit 30 or the measurement item received by the measurement item designation receiving unit 28 (S37), and the measurement point arrangement rule setting unit 33 sets a measurement point arrangement rule based on the measurement item received by the measurement item designation receiving unit 28 (step S38). Then, the measurement unit 31 detects the inside of the detection range set by the detection condition setting unit 32 based on the detection algorithm set by the detection measurement algorithm setting unit 29, and detects the accurate position and region of the measurement target M1 on the ultrasound image Ib. The measurement unit 31 calls, for example, an association table shown in Table 1, which is previously selected by the operator and stored in the image processing and storage unit 26, determines the optimal measurement points K1 and K2 for the detected measurement target M1 based on the called association table, and measures the length of the line K3 connecting the measurement points K1 and K2 to each other (step S39). Then, the measurement unit 31 displays the measurement points K1 and K2 and the line K3, the measurement target and the size or length of the measurement target as the measurement result R, and the like on the image display unit 14 through the image processing and storage unit 26 and the display controller 27 (step S40). As described above, the acoustic wave diagnostic apparatus 1-3 measures the measurement target.

As described above, by setting the measurement point arrangement rule based on the measurement item, the measurement unit 31 can perform measurement at the optimal measurement point according to the measurement item. The acoustic wave diagnostic apparatus 1-3 of the present embodiment is obtained by further providing the measurement point arrangement rule setting unit 33 in the acoustic wave diagnostic apparatus 1-2 shown in Fig. 11. However, the present invention is not limited thereto, and the measurement point arrangement rule setting unit 33 may be provided in the acoustic wave diagnostic apparatus 1 shown in Fig. 10.

Up to now, the embodiments in which only the ultrasound image Ib is displayed as an acoustic wave image in the present invention have been described. However, even in a case where a photoacoustic wave image is displayed as an acoustic wave image, the effect of the present invention can be obtained. In addition, as an acoustic wave image, a composite image obtained by superimposing an ultrasound image and a photoacoustic wave image may be displayed. Also in this case, the effect of the present invention can be obtained.

The acoustic wave diagnostic apparatus according to the embodiment of the present invention is not limited to the embodiments described above, and can be appropriately changed without departing from the spirit of the invention.

### Explanation of References

1: acoustic wave diagnostic apparatus
10: acoustic wave image capturing apparatus
10C: housing
11: probe
12: ultrasound unit
13: laser unit
14: image display unit
15: input unit
15T: touch panel
15B: input button
15K: input key
20: transducer array
21: reception circuit
22: reception memory
23: data separation unit
24: photoacoustic image generation unit
25: ultrasound image generation unit
26: image processing and storage unit
27: display controller
28: measurement item designation receiving unit
29: detection measurement algorithm setting unit
30: position designation receiving unit
31: measurement unit
32: detection condition setting unit
33: measurement point arrangement rule setting unit
34: transmission control circuit
35: controller
40: light emitting unit
50: housing
60: optical fiber
65: absorber
Ib: ultrasound image
L: laser light (measurement light)
M: subject
M1 : measurement target
N: list of measurement items
P: finger of operator

## Claims

1. An acoustic wave diagnostic apparatus, comprising:
an image display unit that displays an acquired acoustic wave image;
a measurement item designation receiving unit that receives designation of a measurement item relevant to a measurement target;
a detection measurement algorithm setting unit that sets a detection measurement algorithm based on the measurement item received by the measurement item designation receiving unit;
a position designation receiving unit that receives designation of a position of the measurement target on the acoustic wave image displayed on the image display unit; and
a measurement unit that, in a case where the designation of the position is received by the position designation receiving unit, detects the measurement target based on the received position and the detection measurement algorithm set by the detection measurement algorithm setting unit and performs measurement for the detected measurement target.

2. The acoustic wave diagnostic apparatus according to claim 1,
wherein the measurement unit determines a position of a detection range in which the detection is performed based on the position received by the position designation receiving unit.

3. The acoustic wave diagnostic apparatus according to claim 1 or 2,
wherein the measurement unit determines a measurement position at which the measurement is performed based on the position received by the position designation receiving unit.

4. The acoustic wave diagnostic apparatus according to any one of claims 1 to 3, further comprising:
a detection condition setting unit that sets condition, under which the detection for the measurement target is performed, based on at least one of the measurement item or the position received by the position designation receiving unit,
wherein the measurement unit performs detection based on the condition set by the detection condition setting unit.

5. The acoustic wave diagnostic apparatus according to claim 4,
wherein the detection condition setting unit sets at least one of a position of a detection range, a size of the detection range, a detection accuracy, or a detection order as the condition for performing the detection.

6. The acoustic wave diagnostic apparatus according to any one of claims 1 to 5, further comprising:
a measurement point arrangement rule setting unit that sets an arrangement rule of measurement points in the measurement target based on the measurement item,
wherein the measurement unit performs measurement by arranging measurement points based on the measurement point arrangement rule set by the measurement point arrangement rule setting unit.

7. The acoustic wave diagnostic apparatus according to any one of claims 1 to 6,
wherein the measurement item designation receiving unit receives designation of at least one of a name of an organ, a name of a lesion, a name of an organ and measurement content regarding the organ, or a name of a lesion and measurement content regarding the lesion as a measurement item relevant to the measurement target.

8. The acoustic wave diagnostic apparatus according to any one of claims 1 to 7,
wherein the acoustic wave image is an ultrasound image.

9. The acoustic wave diagnostic apparatus according to any one of claims 1 to 7,
wherein the acoustic wave image is a photoacoustic wave image.

10. An operation method of an acoustic wave diagnostic apparatus comprising an image display unit, a measurement item designation receiving unit, a detection measurement algorithm setting unit, a position designation receiving unit, and a measurement unit, the method comprising:
causing the image display unit to display an acquired acoustic wave image;
causing the measurement item designation receiving unit to receive designation of a measurement item relevant to a measurement target;
causing the detection measurement algorithm setting unit to set a detection measurement algorithm based on the measurement item received by the measurement item designation receiving unit;
causing the position designation receiving unit to receive designation of a position of the measurement target on the acoustic wave image displayed on the image display unit; and
in a case where the designation of the position is received by the position designation receiving unit, causing the measurement unit to detect the measurement target based on the received position and the detection measurement algorithm set by the detection measurement algorithm setting unit and perform measurement for the detected measurement target.
